# EUROPEAN PATENT SPECIFICATION

(11) **EP 2 443 482 B1**
(45) Date of publication and mention of the grant of the patent: **01.03.2017**
(21) Application number: 10724979.9
(22) Date of filing: 14.06.2010
(51) Int. Cl.: G02B 1/04, A61L 27/52

(54) **BIOMEDICAL DEVICES, POLYMERIC MATERIALS AND CONTACT LENSES COMPRISING THE SAME.**
BIOMEDIZINISCHE VORRICHTUNGEN, POLYMERE MATERIALEN UND KONTAKTLINSEN UMFASSEND DIESELBEN.
DISPOSITIFS BIOMÉDICAUX, MATERIAUX POLYMÈRES ET LENTILLES DE CONTACT LES COMPRENANT.

(30) Priority: 16.06.2009 US 456422
(43) Date of publication of application: 25.04.2012
(73) Proprietor: Bausch & Lomb Incorporated, Rochester, NY 14609 (US)
(72) Inventor: NUNEZ, Ivan, M., Penfield, NY 14526 (US); LINHARDT, Jeffrey, G., Fairport, NY 14450 (US); MCGEE, Joseph, A., Canandaigua, NY 14424 (US); HUNT, Jennifer, Batavia, NY 14020 (US); ALTON, Michele, Rochester, NY 14610 (US); SHIPP, Devon, A., Potsdam, NY 13676 (US); KUNZLER, Jay, Friedrich, Canandaigua, NY 14424 (US); AMMON, Daniel, M., Webster, NY 14580 (US)
(74) Representative: Glas, Holger
(86) International application number: PCT/US2010/038447
(87) International publication number: WO 2010/147864

(56) References cited:
- WO-A1-2008/124093
- WO-A1-2009/085756
- WO-A1-2009/085759
- LOWE ET AL: "Reversible addition-fragmentation chain transfer (RAFT) radical polymerization and the synthesis of water-soluble (co)polymers under homogeneous conditions in organic and aqueous media", PROGRESS IN POLYMER SCIENCE, PERGAMON PRESS, OXFORD, GB, vol. 32, no. 3, 2 March 2007 (2007-03-02), pages 283-351, XP005911232, ISSN: 0079-6700, DOI: DOI:10.1016/J.PROGPOLYMSCI.2006.11.003
- PAVLOVIC D ET AL: "Synthesis of amphiphilic multiblock and triblock copolymers of polydimethylsiloxane and poly(N,N-dimethylacrylamide)", JOURNAL OF POLYMER SCIENCE. PART A, POLYMER CHEMISTRY, JOHN WILEY & SONS, INC, US, vol. 46, no. 21, 1 November 2008 (2008-11-01), pages 7033-7048, XP002537458, ISSN: 0887-624X, DOI: DOI:10.1002/POLA.23009
- PAI T S C ET AL: "Synthesis of amphiphilic block copolymers based on poly(dimethylsiloxane) via fragmentation chain transfer (RAFT) polymerization", POLYMER, ELSEVIER SCIENCE PUBLISHERS B.V, GB, vol. 45, no. 13, 1 June 2004 (2004-06-01), pages 4383-4389, XP004509958, ISSN: 0032-3861, DOI: DOI:10.1016/J.POLYMER.2004.04.041

## Description

### BACKGROUND OF THE INVENTION

### 1. Technical Field

The present invention generally relates to hydrogel contact lenses.

### 2. Description of Related Art

Biomedical devices such as contact lenses are made of various polymeric materials, including rigid gas permeable materials, soft elastomeric materials, and soft hydrogel materials. The majority of contact lenses sold today are made of soft hydrogel materials. Hydrogels are a cross-linked polymeric system that absorb and retain water, typically 10 to 80 percent by weight, and especially 20 to 70 percent water. Hydrogel lenses are commonly prepared by polymerizing a lens-forming monomer mixture including at least one hydrophilic monomer, such as 2-hydroxyethyl methacrylate, N,N-dimethylacrylamide, N-vinyl-2-pyrrolidone, glycerol methacrylate, and methacrylic acid. In the case of silicone hydrogel lenses, a silicone-containing monomer is copolymerized with the hydrophilic monomers. Regardless of their water content, both hydrogel and non-hydrogel siloxy and/or fluorinated contact lenses tend to have relatively hydrophobic, non-wettable surfaces.

The article "Synthesis of Amphiphilic Multiblock and Triblock Copolymers of Polydimethylsiloxane and Poly(N,N-Dimethylacrylamide)" by D. Pavlovic et al., Journal of Polymer Science, Part A, Vol. 46, 7033 - 7048, 2008 refers to the synthesis and the spectroscopic characterization of amphiphilic multiblock and triblock copolymers. The article "Synthesis of amphiphilic block copolymers based on poly(dimethylsiloxane) via fragmentation chain transfer (RAFT) polymerization" by T. Pai et al., Polymer, Vol. 45, 4383 - 4389, 2004 refers to the preparation of A-B-A triblock macromolecules by growing two statistical copolymer blocks from a central PDMS block, comprising units of DMA and BFA.

WO 2009/085759 refers to segmented reactive block copolymers. The reactive segmented block copolymers comprise a chemical binding unit block and a hydrophilic block and are useful as surface coatings for ophthalmic devices. WO 2009/085756 refers to coating solutions comprising segmented interactive block copolymers. The segmented interactive block copolymers in the coating solution can attache to the surface of a substrate by means of interactive functionalities.

In the field of biomedical devices such as contact lenses, various physical and chemical properties such as, for example, oxygen permeability, wettability, material strength and stability are but a few of the factors that must be carefully balanced in order to provide a useable contact lens. For example, since the cornea receives its oxygen supply from contact with the atmosphere, good oxygen permeability is an important characteristic for certain contact lens material. Wettability also is important in that, if the lens is not sufficiently wettable, it does not remain lubricated and therefore cannot be worn comfortably in the eye. Accordingly, the optimum contact lens would have at least both excellent oxygen permeability and excellent tear fluid wettability.

One problem associated with silicone lenses is the surfacing of silicone chains which create hydrophobic areas on the lens. This will adversely impact wettability, on eye-movement and comfort to the user.

One way to alleviate this problem is by coating the surface of silicone hydrogel contact lenses with hydrophilic coatings, such as plasma coatings.

It would be desirable to provide improved biomedical devices such as contact lenses that exhibit suitable physical and chemical properties, e.g., oxygen permeability, lubriciousness and wettability, for prolonged contact with the body while also being biocompatible. It would also be desirable to provide improved biomedical devices that are easy to manufacture in a simple, cost effective manner.

### SUMMARY OF THE INVENTION

In accordance with one embodiment of the present invention, a hydrogel contact lens is provided formed from a free radical polymerization product of a mixture comprising (a) one or more hydrophilic polymers comprising hydrophilic units and one or more thio carbonyl thio fragments of a reversible addition fragmentation chain transfer ("RAFT") agent; (b) one or more silicone-containing monomers, and (c) a polymerization initiator, wherein the hydrophilic units are derived from a hydrophilic monomer selected from the group consisting of an unsaturated carboxylic acid, acrylamide, vinyl lactam, poly(alkyleneoxy)(meth)acrylate, (meth)acrylic acid, hydroxyl-containing-(meth)acrylate, hydrophilic vinyl carbonate, hydrophilic vinyl carbamate monomer, hydrophilic oxazolone monomer, and mixtures thereof and wherein the one or more thio carbonyl thio fragments is of a RAFT agent having the general formula 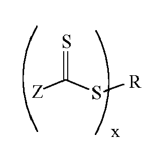 wherein x is 1 or 2, Z is a substituted oxygen, a substituted nitrogen, a substituted sulfur, a substituted or unsubstituted C₁-C₂₀ alkyl or C₃-C₂₅ unsaturated, or partially or fully saturated ring or a carboxylic acid-containing group; and R is independently a straight or branched, substituted or unsubstituted C₁-C₃₀ alkyl group, a substituted or unsubstituted C₃-C₃₀ cycloalkyl group, a substituted or unsubstituted C₃-C₃₀ cycloalkylalkyl group, a substituted or unsubstituted C₃-C₃₀ cycloalkenyl group, a substituted or unsubstituted C₅-C₃₀ aryl group, a substituted or unsubstituted C₅-C₃₀ arylalkyl group, a C₁-C₂₀ ester group; an ether or polyether-containing group; an alkyl- or arylamide group; an alkyl- or arylamine group; a substituted or unsubstituted C₅-C₃₀ heteroaryl group; a substituted or unsubstituted C₃-C₃₀ heterocyclic ring; a substituted or unsubstituted C₄-C₃₀ heterocycloalkyl group; a substituted or unsubstituted C₆-C₃₀ heteroarylalkyl group; and combinations thereof The hydrophilic polymers containing one or more hydrophilic units and one or more thio carbonyl thio fragments of a RAFT agent are non-amphophilic polymers and are capable of forming biomedical devices with a hydrophilic or lubricious (or both) surface. Hydrophilic and/or lubricious surfaces of the biomedical devices herein such as contact lenses substantially prevent or limit the adsorption of tear lipids and proteins on, and their eventual absorption into, the lenses, thus preserving the clarity of the contact lenses. This, in turn, preserves their performance quality thereby providing a higher level of comfort to the wearer.

### DETAILED DESCRIPTION OF THE PREFERRED EMBODIMENTS

The present invention is directed to a biomedical device, namely a hydrogel contact lens intended for direct contact with body tissue or body fluid. A "biomedical device" is any article that is designed to be used while either in or on mammalian tissues or fluid, and preferably in or on human tissue or fluids. Representative examples of biomedical devices include, but are not limited to, artificial ureters, diaphragms, intrauterine devices, heart valves, catheters, denture liners, prosthetic devices, ophthalmic lens applications, where the lens is intended for direct placement in or on the eye, such as, for example, intraocular devices and contact lenses. The preferred biomedical devices are ophthalmic devices, particularly contact lenses, and most particularly contact lenses made from hydrogels.

An "ophthalmic device" refers to devices that reside in or on the eye. These devices can provide optical correction, wound care, drug delivery, diagnostic functionality or cosmetic enhancement or effect or a combination of these properties. Useful ophthalmic devices include, but are not limited to, ophthalmic lenses such as soft contact lenses, e.g., a soft, hydrogel lens, soft, non-hydrogel lens and the like, hard contact lenses, e.g., a hard, gas permeable lens material and the like, intraocular lenses, overlay lenses, ocular inserts, optical inserts and the like. As is understood by one skilled in the art, a lens is considered to be "soft" if it can be folded back upon itself without breaking. The present invention refers to a hydrogel contact lens.

The hydrogel contact lenses of the present invention are formed from a free radical polymerization product of a mixture according to claim 1.

The one or more hydrophilic polymers comprising one or more hydrophilic units and one or more thio carbonyl thio fragments of a RAFT agent are prepared via RAFT polymerization, i.e., monomers are polymerized via a RAFT mechanism to form the hydrophilic polymer, e.g., a block or random copolymer in which the molecular weight of each of the blocks and the entire polymer can be precisely controlled. Thus, RAFT polymerization is a radical polymerization technique that enables polymers to be prepared having a well defined molecular architecture and low polydispersity.

The RAFT agents suitable for use herein are based upon thio carbonyl thio chemistry which is well known to those of ordinary skill in the art. The RAFT agent can be, for example, a xanthate-containing compound, trithiocarbonate-containing compound, dithiocarbamate-containing compound or dithio ester-containing compound, wherein each compound contains a thiocarbonyl thio group. The class of RAFT agents that is used herein is of the general formula: 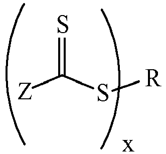 wherein x is 1 or 2, Z is a substituted oxygen (e.g., xanthates (-O-R)), a substituted nitrogen (e.g., dithiocarbamates (-NRR)), a substituted sulfur (e.g., trithiocarbonates (-S-R)), a substituted or unsubstituted C₁-C₂₀ alkyl or C₃-C₂₅ unsaturated, or partially or fully saturated ring (e.g., dithioesters (-R)) or a carboxylic acid-containing group; and R is independently a straight or branched, substituted or unsubstituted C₁-C₃₀ alkyl group, a substituted or unsubstituted C₃-C₃₀ cycloalkyl group, a substituted or unsubstituted C₃-C₃₀ cycloalkylalkyl group, a substituted or unsubstituted C₃-C₃₀ cycloalkenyl group, a substituted or unsubstituted C₅-C₃₀ aryl group, a substituted or unsubstituted C₅-C₃₀ arylalkyl group, a C₁-C₂₀ ester group; an ether or polyether-containing group; an alkyl- or arylamide group; an alkyl- or arylamine group; a substituted or unsubstituted C₅-C₃₀ heteroaryl group; a substituted or unsubstituted C₃-C₃₀ heterocyclic ring; a substituted or unsubstituted C₄-C₃₀ heterocycloalkyl group; a substituted or unsubstituted C₆-C₃₀ heteroarylalkyl group; and combinations thereof.

Representative examples of alkyl groups for use herein include, by way of example, a straight or branched alkyl chain radical containing carbon and hydrogen atoms of from 1 to about 30 carbon atoms and preferably from 1 to about 12 carbon atoms with or without unsaturation, to the rest of the molecule, e.g., methyl, ethyl, n-propyl, 1-methylethyl (isopropyl), n-butyl, n-pentyl, methylene, ethylene, etc., and the like.

Representative examples of cycloalkyl groups for use herein include, by way of example, a substituted or unsubstituted non-aromatic mono or multicyclic ring system of about 3 to about 30 carbon atoms and preferably from 3 to about 6 carbon atoms such as, for example, cyclopropyl, cyclobutyl, cyclopentyl, cyclohexyl, perhydronapththyl, adamantyl and norbornyl groups, bridged cyclic groups or sprirobicyclic groups, e.g., spiro-(4, 4)-non-2-yl and the like, optionally containing one or more heteroatoms, e.g., O and N, and the like.

Representative examples of cycloalkylalkyl groups for use herein include, by way of example, a substituted or unsubstituted cyclic ring-containing radical containing from about 3 to about 30 carbon atoms and preferably from 3 to about 6 carbon atoms directly attached to the alkyl group which are then attached to the main structure of the monomer at any carbon from the alkyl group that results in the creation of a stable structure such as, for example, cyclopropylmethyl, cyclobutylethyl, cyclopentylethyl and the like, wherein the cyclic ring can optionally contain one or more heteroatoms, e.g., O and N, and the like.

Representative examples of cycloalkenyl groups for use herein include, by way of example, a substituted or unsubstituted cyclic ring-containing radical containing from about 3 to about 30 carbon atoms and preferably from 3 to about 6 carbon atoms with at least one carbon-carbon double bond such as, for example, cyclopropenyl, cyclobutenyl, cyclopentenyl and the like, wherein the cyclic ring can optionally contain one or more heteroatoms, e.g., O and N, and the like.

Representative examples of aryl groups for use herein include, by way of example, a substituted or unsubstituted monoaromatic or polyaromatic radical containing from about 5 to about 30 carbon atoms such as, for example, phenyl, naphthyl, tetrahydronapthyl, indenyl, biphenyl and the like, optionally containing one or more heteroatoms, e.g., O and N, and the like.

Representative examples of arylalkyl groups for use herein include, by way of example, a substituted or unsubstituted aryl group as defined herein directly bonded to an alkyl group as defined herein, e.g., -CH₂C₆H₅, -C₂H₅C₆H₅ and the like, wherein the aryl group can optionally contain one or more heteroatoms, e.g., O and N, and the like.

Representative examples of ester groups for use herein include, by way of example, a carboxylic acid ester having one to 20 carbon atoms and the like.

Representative examples of ether or polyether containing groups for use herein include, by way of example, an alkyl ether, cycloalkyl ether, cycloalkylalkyl ether, cycloalkenyl ether, aryl ether, arylalkyl ether wherein the alkyl, cycloalkyl, cycloalkylalkyl, cycloalkenyl, aryl, and arylalkyl groups are as defined herein. Exemplary ether or polyether-containing groups include, by way of example, alkylene oxides, poly(alkylene oxide)s such as ethylene oxide, propylene oxide, butylene oxide, poly(ethylene oxide)s, poly(ethylene glycol)s, poly(propylene oxide)s, poly(butylene oxide)s and mixtures or copolymers thereof, an ether or polyether group of the general formula -(R²OR³)ₜ, wherein R² is a bond, a substituted or unsubstituted alkyl, cycloalkyl or aryl group as defined herein and R³ is a substituted or unsubstituted alkyl, cycloalkyl or aryl group as defined herein and t is at least 1, e.g., -CH₂CH₂OC₆H₅ and CH₂-CH₂-CH₂-O-CH₂-(CF₂)_{z}-H where z is 1 to 6, -CH₂CH₂OC₂H₅, and the like.

Representative examples of alkyl or arylamide groups for use herein include, by way of example, an amide of the general formula -R⁴C(O)NR⁵R⁶ wherein R⁴, R⁵ and R⁶ are independently C₁-C₃₀ hydrocarbons, e.g., R⁴ can be alkylene groups, arylene groups, cycloalkylene groups and R⁵ and R⁶ can be alkyl groups, aryl groups, and cycloalkyl groups as defined herein and the like.

Representative examples of alky or arylamine groups for use herein include, by way of example, an amine of the general formula -R⁷N R⁸R⁹ wherein R⁷ is a C₂-C₃₀ alkylene, arylene, or cycloalkylene and R⁸ and R⁹ are independently C₁-C₃₀ hydrocarbons such as, for example, alkyl groups, aryl groups, or cycloalkyl groups as defined herein.

Representative examples of heterocyclic ring groups for use herein include, by way of example, a substituted or unsubstituted stable 3 to about 30 membered ring radical, containing carbon atoms and from one to five heteroatoms, e.g., nitrogen, phosphorus, oxygen, sulfur and mixtures thereof. Suitable heterocyclic ring radicals for use herein may be a monocyclic, bicyclic or tricyclic ring system, which may include fused, bridged or spiro ring systems, and the nitrogen, phosphorus, carbon, oxygen or sulfur atoms in the heterocyclic ring radical may be optionally oxidized to various oxidation states. In addition, the nitrogen atom may be optionally quaternized; and the ring radical may be partially or fully saturated (i.e., heteroaromatic or heteroaryl aromatic). Examples of such heterocyclic ring radicals include, but are not limited to, azetidinyl, acridinyl, benzodioxolyl, benzodioxanyl, benzofurnyl, carbazolyl, cinnolinyl, dioxolanyl, indolizinyl, naphthyridinyl, perhydroazepinyl, phenazinyl, phenothiazinyl, phenoxazinyl, phthalazinyl, pyridyl, pteridinyl, purinyl, quinazolinyl, quinoxalinyl, quinolinyl, isoquinolinyl, tetrazoyl, imidazolyl, tetrahydroquinolinyl, tetrahydroisoquinolinyl, piperidinyl, piperazinyl, 2-oxopiperazinyl, 2-oxopiperidinyl, 2-oxopyrrolidinyl, 2-oxoazepinyl, azepinyl, pyrrolyl, 4-piperidonyl, pyrrolidinyl, pyrazinyl, pyrimidinyl, pyridazinyl, oxazolyl, oxazolinyl, oxazolidinyl, triazolyl, indanyl, isoxazolyl, iso-oxazolidinyl, morpholinyl, thiazolyl, thiazolinyl, thiazolidinyl, isothiazolyl, quinuclidinyl, isothiazolidinyl, indolyl, isoindolyl, indolinyl, isoindolinyl, octahydroindolyl, octahydroisoindolyl, quinolyl, isoquinolyl, decahydroisoquinolyl, benzimidazolyl, thiadiazolyl, benzopyranyl, benzothiazolyl, benzooxazolyl, furyl, tetrahydrofurtyl, tetrahydropyranyl, thienyl, benzothienyl, thiamorpholinyl, thiamorpholinyl sulfoxide, thiamorpholinyl sulfone, dioxaphospholanyl, oxadiazolyl, chromanyl, isochromanyl and the like and mixtures thereof.

Representative examples of heteroaryl groups for use herein include, by way of example, a substituted or unsubstituted heterocyclic ring radical as defined herein. The heteroaryl ring radical may be attached to the main structure at any heteroatom or carbon atom that results in the creation of a stable structure.

Representative examples of heteroarylalkyl groups for use herein include, by way of example, a substituted or unsubstituted heteroaryl ring radical as defined herein directly bonded to an alkyl group as defined herein. The heteroarylalkyl radical may be attached to the main structure at any carbon atom from the alkyl group that results in the creation of a stable structure.

Representative examples of heterocyclic groups for use herein include, by way of example, a substituted or unsubstituted heterocylic ring radical as defined herein. The heterocyclic ring radical may be attached to the main structure at any heteroatom or carbon atom that results in the creation of a stable structure.

Representative examples of heterocycloalkyl groups for use herein include, by way of example, a substituted or unsubstituted heterocylic ring radical as defined herein directly bonded to an alkyl group as defined herein. The heterocycloalkyl radical may be attached to the main structure at any carbon atom in the alkyl group that results in the creation of a stable structure.

The substituents in the 'substituted oxygen', 'substituted nitrogen', 'substituted sulfur', 'substituted alkyl', 'substituted alkylene, 'substituted cycloalkyl', 'substituted cycloalkylalkyl', 'substituted cycloalkenyl', 'substituted arylalkyl', 'substituted aryl', 'substituted heterocyclic ring', 'substituted heteroaryl ring,' 'substituted heteroarylalkyl', 'substituted heterocycloalkyl ring', 'substituted cyclic ring' may be the same or different and include one or more substituents such as hydrogen, hydroxy, halogen, carboxyl, cyano, nitro, oxo (=O), thio(=S), substituted or unsubstituted alkyl, substituted or unsubstituted alkoxy, substituted or unsubstituted alkenyl, substituted or unsubstituted alkynyl, substituted or unsubstituted aryl, substituted or unsubstituted arylalkyl, substituted or unsubstituted cycloalkyl, substituted or unsubstituted cycloalkenyl, substituted or unsubstituted amino, substituted or unsubstituted aryl, substituted or unsubstituted heteroaryl, substituted heterocycloalkyl ring, substituted or unsubstituted heteroarylalkyl, substituted or unsubstituted heterocyclic ring, and the like.

In one embodiment, x is 2 and R is an ether or polyether containing group as defined above. For example, the RAFT agent can be prepared according to the following general scheme:

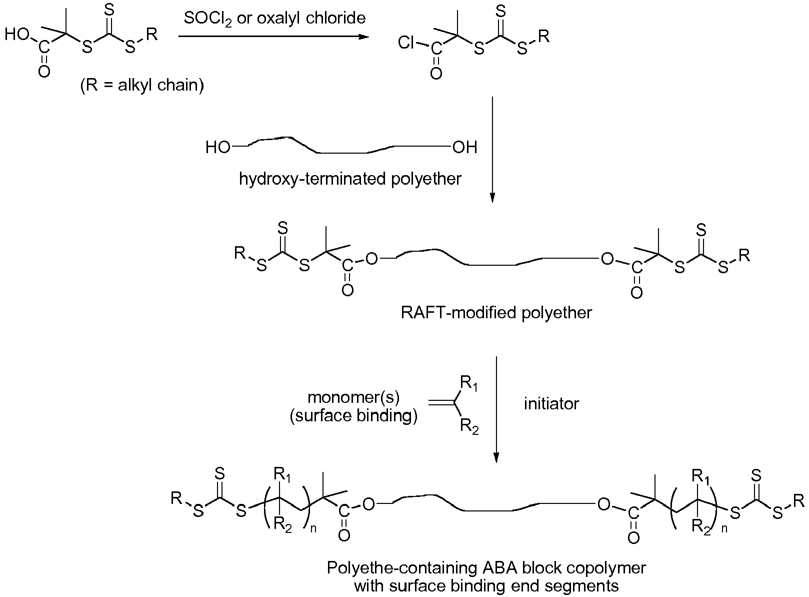

Another class of known RAFT agents is of the general formula: 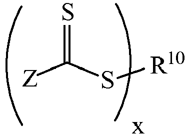 wherein x and Z have the aforestated meanings and R¹⁰ is a substituted or unsubstituted carboxylic acid-containing group.

Representative examples of a carboxylic acid-containing group include, by way of example, a carboxylic acid group attached to the rest of the molecule via a linking group, e.g., of the general formula -R¹¹C(O)OH, wherein R¹¹ is a bond, a substituted or unsubstituted alkylene group, a substituted or unsubstituted cycloalkylene group, a substituted or unsubstituted cycloalkylalkylene group, a substituted or unsubstituted arylene or a substituted or unsubstituted arylalkylene group as defined herein, e.g., -CH(Ar)(C(O)OH), -C(CH₃)(C(O)OH), and the like, wherein the carboxylic acid group can be attached to the substituent or attached directly to alkylene group, cycloalkylene group, cycloalkylalkylene group, arylene or arylalkylene group.

Representative examples of RAFT agents for use herein include, but are not limited to, benzyl dodecyl trithiocarbonate, ethyl-2-dodecyl trithiocarbony) proprionate, S-sec propionic acid O-ethyl xanthate, α-ethyl xanthylphenylacetic acid, ethyl α-(o-ethyl xanthyl) proprionate, ethyl α-(ethyl xanthyl) phenyl acetate, ethyl 2-(dodecyl trithiocarbonyl) phenyl acetate, ethyl 2-(dodecyl trithiocarbonyl) propionate, 2-(dodecylthiocarbonylthiol)propanoic acid, and the like and mixtures thereof.

There is no particular limitation on the organic chemistry used to form the RAFT agent and is within the purview of one skilled in the art. Also, the working examples below provide guidance. For example, the RAFT agents can be prepared as exemplified in Schemes I-III below.

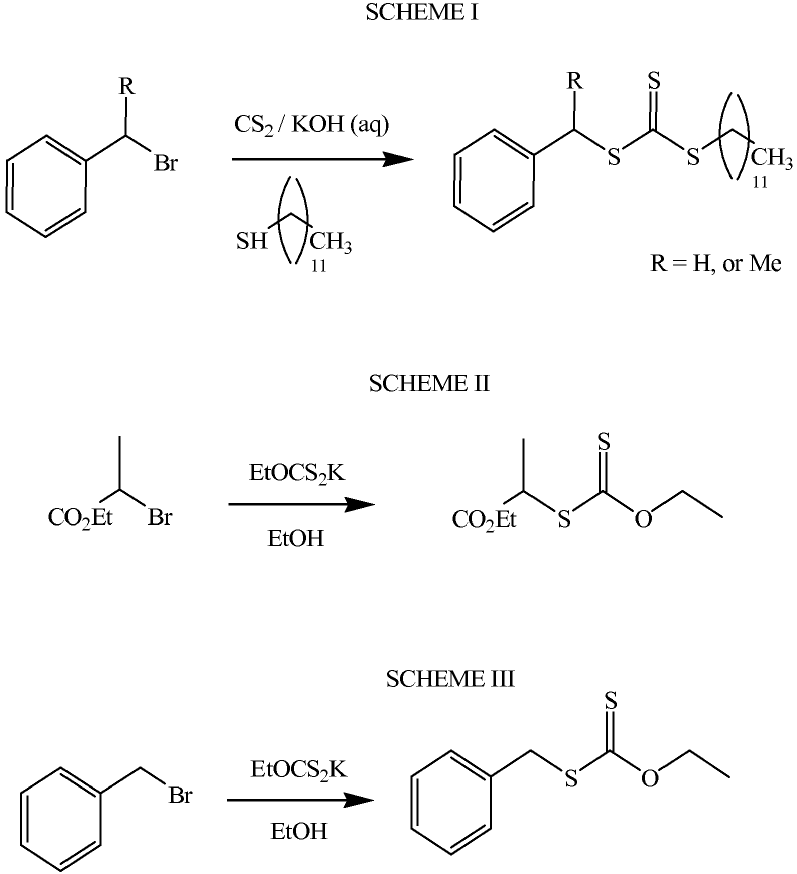

In addition to the one or more thio carbonyl thio fragments of a RAFT agent, the hydrophilic polymers described herein also contain one or more hydrophilic units. In general, the hydrophilic unit(s) is derived from at least one hydrophilic monomer. Suitable hydrophilic monomer include, acrylamides such as N,N-dimethylacrylamide, N,N-dimethylmethacrylamide, and the like; vinyl lactams such as N-vinyl-2-pyrrolidone and the like; (meth)acrylated alcohols such as 2-hydroxyethyl methacrylate, 2-hydroxyethyl acrylate and the like; ethylenically unsaturated carboxylic acids such as methacrylic acid, acrylic acid and the like and mixtures thereof.

Further described herein are hydrophilic polymers containing one or more thio carbonyl thio fragments of a RAFT agent that include a hydrophilic unit derived from an ethylenically unsaturated polymerizable monomer having ring-opening reactive functionalities. Such monomers may include one or more ring-opening reactive groups such as, for example, azlactone, epoxy, acid anhydrides, and the like. Suitable polymerizable monomer having ring-opening reactive functionalities include, but are not limited to, glycidyl methacrylate (GMA), maleic anhydride, itaconic anhydride and the like and mixtures thereof. The units derived from an ethylenically unsaturated polymerizable monomer having ring-opening reactive functionalities can be copolymerized with a hydrophilic comonomer to form the hydrophilic units in the resulting hydrophilic polymers. Non-limiting examples of comonomers useful to be copolymerized with the ring-opening reactive functionalities of the monomer to form hydrophilic polymers used to prepare a biomedical device include those mentioned above, with dimethylacrylamide, hydroxyethyl methacrylate (HEMA), and/or N-vinylpyrrolidone being preferred. Alternatively, the unit derived from the ethylenically unsaturated polymerizable hydrophilic monomers having ring-opening reactive functionalities can be subjected to a ring-opening reaction, e.g., by hydrolyzing with water, and form hydrophilic units in the resulting hydrophilic polymer.

In one embodiment, the hydrophilic polymers according to claim 1 can also include a unit derived from an ethylenically unsaturated polymerizable alkoxylated polymer. Suitable ethylenically unsaturated polymerizable alkoxylated polymers include, by way of example, polymerizable polyethylene glycols having a molecular weight of up to, for example, about 1000 such as those with CTFA names PEG-200, PEG-400, PEG-600, PEG-1000, and mixtures thereof. Representative examples include PEG-200 methacrylate, PEG-400 methacrylate, PEG-600 methacrylate, PEG-1000 methacrylate and the like and mixtures thereof.

The size of the units derived from an ethylenically unsaturated polymerizable alkoxylated polymer can vary widely, e.g., the number of units can range from 2 to about 225, and preferably from about 5 to about 25.

In one embodiment, the hydrophilic polymers containing one or more thio carbonyl thio fragments of a RAFT agent can also include a unit derived from a protected monomer such as, for example, nitrogen protected monomers, acetate protected monomers, e.g., vinyl acetate, and the like. In general, nitrogen protected monomers ("NPM") have an amino group that is protected by a nitrogen protecting group. As used herein, the term "nitrogen protecting group" means a group attached to a nitrogen atom to preclude that nitrogen atom from participating in a polymerization reaction. Although secondary amine groups can be protected in accordance with the invention, in most embodiments the protected amino group provides a primary amine group following deprotection.

The nitrogen protecting groups according to the present invention are "carbamate-type" groups of the formula C(O)O-R', wherein R' is an aromatic or aliphatic hydrocarbon group, which may be optionally substituted and which, taken together with the nitrogen atom to which it is attached forms a carbamate group; Representative examples of nitrogen protecting groups include, but are not limited to, benzyloxycarbonyl (CBZ), p-methoxybenzyloxycarbonyl, p-nitrobenzyloxycarbonyl, tert-butoxycarbonyl (t-BOC), 9-flourenylmethyloxycarbonyl (Fmoc), 2-chlorobenzyloxycarbonyl, allyloxycarbonyl (alloc), 2-(4-biphenylyl)propyl-2-oxycarbonyl (Bpoc), and the like.

In one embodiment, examples of t-Boc protected monomers include 2-(2-(tert-butoxycarbonylamino)acetoxy)ethyl methacrylate, 2-(2-(tert-butoxycarbonylamino)acetamido)ethyl methacrylate, 2-(tert-butoxycarbonylamino)ethyl methacrylate, tert-butyl 2-(vinyloxycarbonyloxy)ethylcarbamate, 2-(tert-butoxycarbonylamino)ethyl-N-vinylcarbamate, 3-(2-(tert-butoxycarbonylamino)acetoxy)-2-hydroxypropyl , N-(tert-Butoxycarbonyl)-L-glutamic acid methacryloxyethyl ester, 2-(tert-butoxycarbonylamino)-6-(3-(2-(methacryloyloxy)ethyl)ureido)hexanoic acid, 2-(tert-butoxycarbonylamino)-3-(methacryloyloxy)propanoic acid, 2-(tert-butoxycarbonylamino)-6-methacrylamidohexanoic acid and the like.

The nitrogen protecting groups present in the hydrophilic polymer can be readily removed post-polymerization by well known methods in the chemical art. Techniques for protecting amino nitrogen atoms with nitrogen protecting groups, and for deprotecting amino nitrogen atoms after a particular reaction are well known in the chemical art. See, for example, Greene et al., Protective Groups in Organic Synthesis, John Wiley & Sons, 1991, and U.S. Provisional Serial Nos. 61/113,736; 61/113,739; 61/113,742; and 61/113,746, the contents of which are incorporated by reference herein. By way of example, an NPM can be prepared by reaction of a nitrogen-protected amino acid or amino alcohol with an ethylenically unsaturated compound having a group reactive with the respective acid or an alcohol group. In some embodiments a nitrogen protected amino acid may also have an unprotected amine group or a hydroxyl group, and the second amine group or the hydroxyl group, respectively, is the site of reaction to attach the ethylenic unsaturation. If the nitrogen protected amino acid has multiple available sites of attachment of an ethylenically unsaturated group NPM monomers having two or more ethylenically unsaturated groups may be produced.

As one skilled in the art will readily understand, these monomers are usually hydrophobic in the "protected" or "blocked" form. In order to become more polar and hydrophilic, the protecting group (e.g., in the case of the t-Boc monomers) will need to be removed from the unit. This will result in the hydrogel contact lens becoming more hydrophilic in nature and the material could therefore retain more water. Methods for removing the protecting group are within the purview of one skilled in the art.

In general, the size of the hydrophilic units can vary widely, e.g., the number of units can range from 10 to about 3000, and preferably from about 50 to about 1000.

The resulting hydrophilic polymers can be in the form of homopolymers, block copolymers and random copolymers. The hydrophilic polymers will have a number average molecular weight ranging from about 1,000 to about 300,000 and about 10,000 to about 100,000.

Methods for preparing hydrophilic polymers containing one or more thio carbonyl thio fragments of a RAFT agent as described above are within the purview of one skilled in the art. Also, the working examples below provide ample guidance. Representative schemes for preparing the hydrophilic polymers are set forth below in Schemes IV-VI: 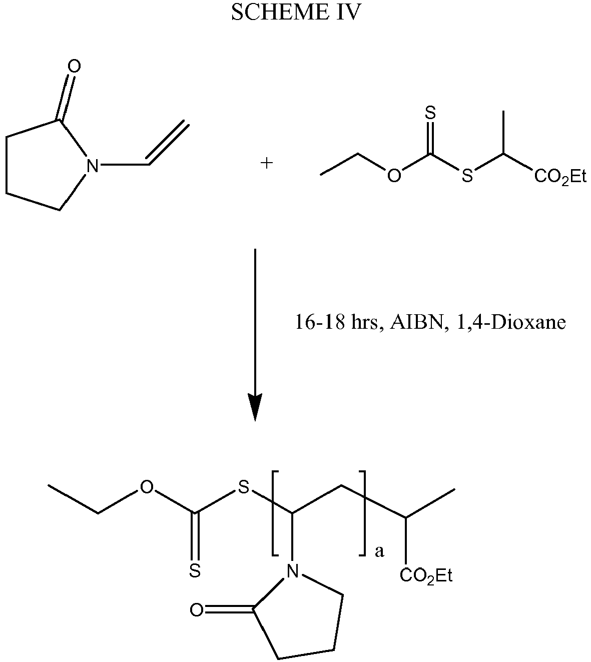 wherein a is from about 10 to about 2,700. 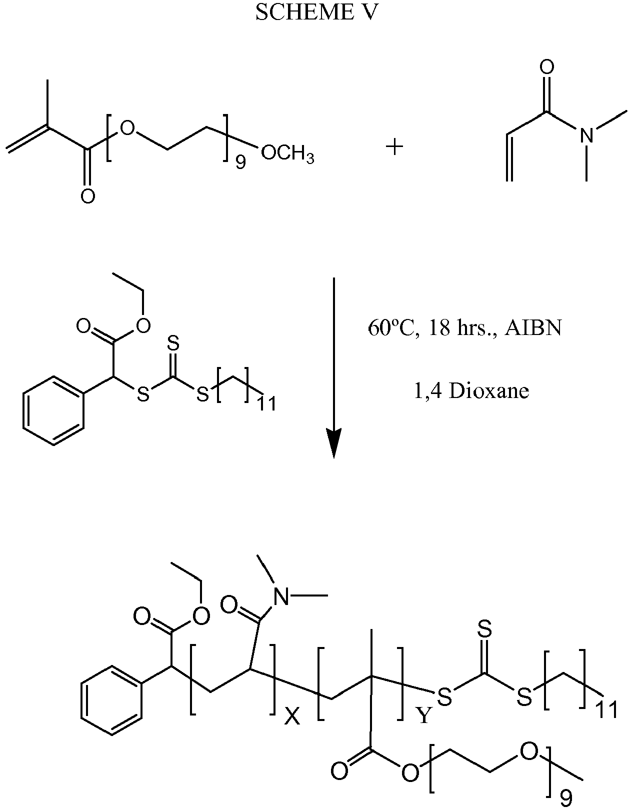 wherein x is from about 15 to about 3000 and y is from about 1 to about 250. 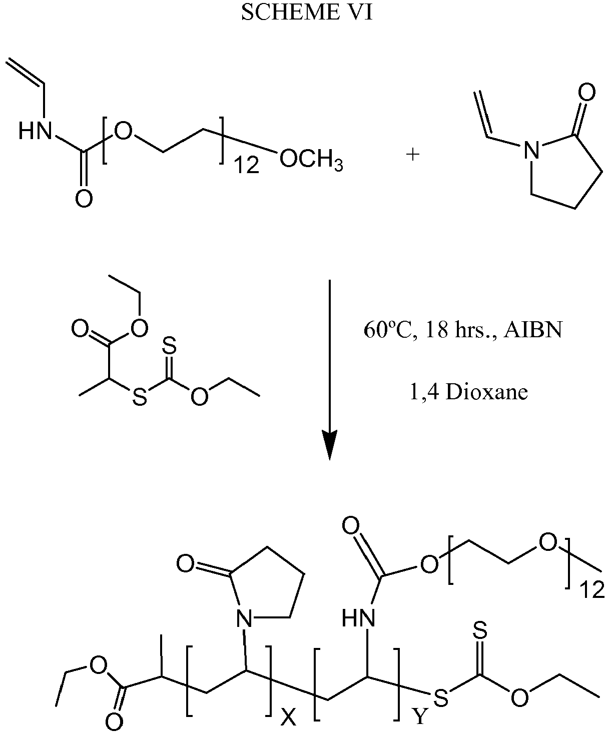 wherein x is from about 12 to about 3000 and y is from about 1 to about 250.

The one or more comonomers employed in the mixtures to be polymerized to form a hydrogel contact lens of the present invention include one or more silicone-containing monomers. As used herein, the term "monomer" or "monomeric" and like terms denote relatively low molecular weight compounds that are polymerizable by free radical polymerization, as well as higher molecular weight compounds also referred to as "prepolymers", "macromonomers", and related terms. Generally, the comonomer contains at least one polymerizable group.

A silicone-containing comonomer which contains from 1 to about 60 silicone atoms, in addition to the hydrophilic polymer containing one or more thio carbonyl thio fragments of a RAFT agent, is included in the initial mixture, for example, if it is desired to obtain a copolymer with high oxygen permeability. Applicable silicone-containing monomers for use in the formation of hydrogel contact lenses such as silicone hydrogels are well known in the art and numerous examples are provided in, for example, U.S. Patent Nos. 4,136,250; 4,153,641; 4,740,533; 5,034,461; 5,070,215; 5,260,000; 5,310,779; and 5,358,995.

Representative examples of applicable silicon-containing monomers include bulky polysiloxanylalkyl(meth)acrylic monomers. An example of a bulky polysiloxanylalkyl(meth)acrylic monomer is represented by the structure of Formula V: 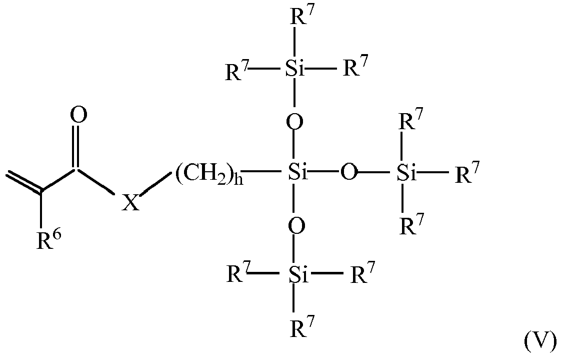 wherein X denotes -O- or -NR- wherein R denotes hydrogen or a C₁-C₄ alkyl; each R⁶ independently denotes hydrogen or methyl; each R⁷ independently denotes a lower alkyl radical, phenyl radical or a group represented by 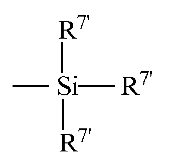 wherein each R^{7'} independently denotes a lower alkyl or phenyl radical; and h is 1 to 10.

Representative examples of other applicable silicon-containing monomers includes, but are not limited to, bulky polysiloxanylalkyl carbamate monomers as generally depicted in Formula Va: 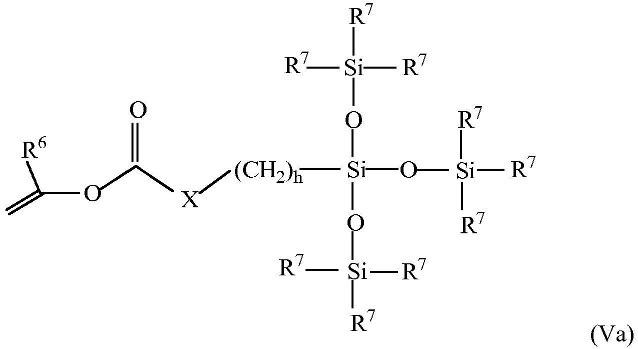 wherein X denotes-NR-; wherein R denotes hydrogen or a C₁-C₄ alkyl; R⁶ denotes hydrogen or methyl; each R⁷ independently denotes a lower alkyl radical, phenyl radical or a group represented by 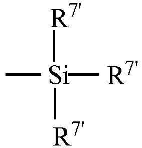 wherein each R^{7'} independently denotes a lower alkyl or phenyl radical; and h is 1 to 10, and the like.

Examples of bulky monomers are 3-methacryloyloxypropyltris(trimethyl-siloxy)silane or tris(trimethylsiloxy)silylpropyl methacrylate, sometimes referred to as TRIS and tris(trimethylsiloxy)silylpropyl vinyl carbamate, sometimes referred to as TRIS-VC and the like and mixtures thereof.

Such bulky monomers may be copolymerized with a silicone macromonomer, which is a poly(organosiloxane) capped with an unsaturated group at two or more ends of the molecule. U.S. Patent No. 4,153,641 discloses, for example, various unsaturated groups such as acryloxy or methacryloxy groups.

Another class of representative silicone-containing monomers includes, but is not limited to, silicone-containing vinyl carbonate or vinyl carbamate monomers such as, for example, 1,3-bis[4-vinyloxycarbonyloxy)but-1-yl]tetramethyldisiloxane; 3-(trimethylsilyl)propyl vinyl carbonate; 3-(vinyloxycarbonylthio)propyl-[tris(trimethylsiloxy)silane]; 3-[tris(trimethylsiloxy)silyl]propyl vinyl carbamate; 3-[tris(trimethylsiloxy)silyl]propyl allyl carbamate; 3-[tris(trimethylsiloxy)silyl]propyl vinyl carbonate; t-butyldimethylsiloxyethyl vinyl carbonate; trimethylsilylethyl vinyl carbonate; trimethylsilylmethyl vinyl carbonate and the like.

Another class of silicon-containing monomers includes polyurethane-polysiloxane macromonomers (also sometimes referred to as prepolymers), which may have hard-soft-hard blocks like traditional urethane elastomers. Examples of silicone urethanes are disclosed in a variety or publications, including Lai, Yu-Chin, "The Role of Bulky Polysiloxanylalkyl Methacrylates in Polyurethane-Polysiloxane Hydrogels," Journal of Applied Polymer Science, Vol. 60, 1193-1199 (1996). PCT Published Application No. WO 96/31792 also discloses examples of such monomers, the contents of which are hereby incorporated by reference in its entirety. Further examples of silicone urethane monomers are represented by Formulae VI and VII:

E(*D*A*D*G)ₐ *D*A*D*E'; or (VI)

E(*D*G*D*A)ₐ *D*A*D*E'; or (VII)

wherein:
D denotes an alkyl diradical, an alkyl cycloalkyl diradical, a cycloalkyl diradical, an aryl diradical or an alkylaryl diradical having 6 to about 30 carbon atoms;
G denotes an alkyl diradical, a cycloalkyl diradical, an alkyl cycloalkyl diradical, an aryl diradical or an alkylaryl diradical having 1 to about 40 carbon atoms and which may contain ether, thio or amine linkages in the main chain;
* denotes a urethane or ureido linkage;
a is at least 1;
A denotes a divalent polymeric radical of Formula VIII: 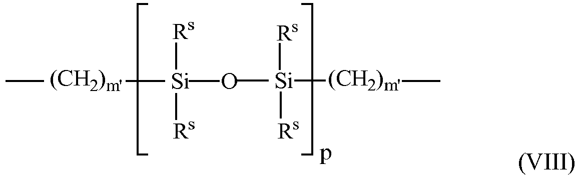 wherein each R^{s} independently denotes an alkyl or fluoro-substituted alkyl group having 1 to about 10 carbon atoms which may contain ether linkages between the carbon atoms; m' is at least 1; and p is a number that provides a moiety weight of about 400 to about 10,000;
each of E and E' independently denotes a polymerizable unsaturated organic radical represented by Formula IX: 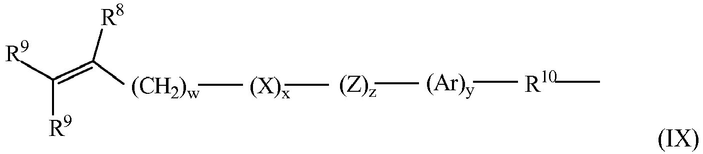
   wherein: R⁸ is hydrogen or methyl;
   R⁹ is independently hydrogen, an alkyl radical having 1 to 6 carbon atoms, or a -CO-Y-R¹¹ radical wherein Y is -O-, -S- or -NH-;
   R¹⁰ is a divalent alkylene radical having 1 to about 10 carbon atoms;
   R¹¹ is a alkyl radical having 1 to about 12 carbon atoms;
   X denotes -CO- or -OCO-;
   Z denotes -O- or -NH-;
   Ar denotes an aromatic radical having about 6 to about 30 carbon atoms;
   w is 0 to 6; x is 0 or 1; y is 0 or 1; and z is 0 or 1.

A preferred silicone-containing urethane monomer is represented by Formula X: 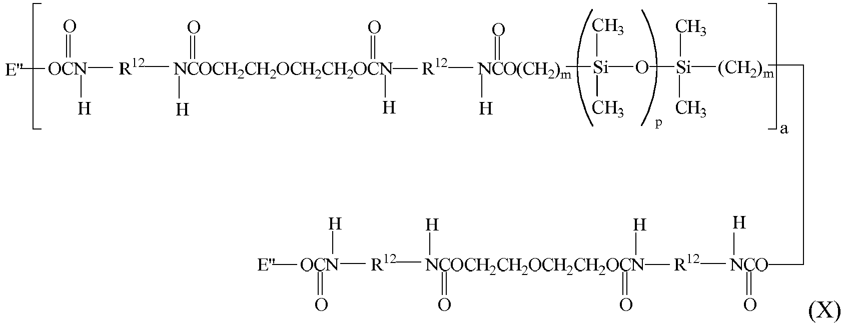 wherein m is at least 1 and is preferably 3 or 4, a is at least 1 and preferably is 1, p is a number which provides a moiety weight of about 400 to about 10,000 and is preferably at least about 30, R¹² is a diradical of a diisocyanate after removal of the isocyanate group, such as the diradical of isophorone diisocyanate, and each E" is a group represented by:

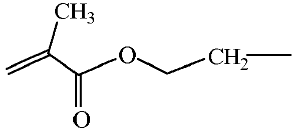

Another class of representative silicone-containing monomers includes fluorinated monomers. Such monomers have been used in the formation of fluorosilicone hydrogels to reduce the accumulation of deposits on contact lenses made therefrom, as described in, for example, U.S. Patent Nos. 4,954,587; 5,010,141 and 5,079,319. The use of silicone-containing monomers having certain fluorinated side groups, i.e., -(CF₂)-H, have been found to improve compatibility between the hydrophilic and silicone-containing monomeric units, see, e.g., U.S. Patent Nos. 5,321,108 and 5,387,662.

The above silicone materials are merely exemplary, and other materials for use in forming biomedical devices according to the present invention and have been disclosed in various publications and are being continuously developed for use in contact lenses and other biomedical devices can also be used. For example, a biomedical device-forming comonomer can be a cationic monomer such as cationic silicone-containing monomer or cationic fluorinated silicone-containing monomers.

The mixtures to be polymerized may include the silicone comonomer, in addition to the subject multi-armed macromonomers, at 0 to about 50 weight percent, preferably about 5 to about 30 weight percent when present.

The mixtures to be polymerized can also include a crosslinking monomer (a crosslinking monomer being defined as a monomer having multiple polymerizable functionalities). Representative crosslinking monomers include: divinylbenzene, allyl methacrylate, ethyleneglycol dimethacrylate, tetraethyleneglycol dimethacrylate, polyethyleneglycol dimethacrylate, vinyl carbonate derivatives of the glycol dimethacrylates, and methacryloxyethyl vinylcarbonate. When a crosslinking agent is employed, this monomeric material may be included in the monomer mixture at about 0.1 to about 20 weight percent, and more preferably at about 0.2 to about 10 weight percent.

Although not necessarily required, homopolymers or copolymers within the scope of the present invention may optionally have one or more strengthening agents added prior to polymerization, preferably in quantities of less than about 80 weight percent and preferably from about 20 to about 60 weight percent. Non-limiting examples of suitable strengthening agents are described in U.S. Patent Nos. 4,327,203; 4,355,147; and 5,270,418; each of which is incorporated herein in its entirety by reference. Specific examples, not intended to be limiting, of such strengthening agents include cycloalkyl acrylates and methacrylates; e.g., tert-butylcyclohexyl methacrylate and isopropylcyclopentyl acrylate.

The mixtures to be polymerized may further contain, as necessary and within limits not to impair the purpose and effect of the present invention, various additives such as an antioxidant, coloring agent, ultraviolet absorber, lubricant internal wetting agents, toughening agents and the like and other constituents as is well known in the art.

The hydrogel contact lenses of the present invention, can be prepared by polymerizing the foregoing mixtures to form a product that can be subsequently formed into the appropriate shape by, for example, lathing, injection molding, compression molding, cutting and the like. For example, in producing contact lenses, the initial mixture may be polymerized in tubes to provide rod-shaped articles, which are then cut into buttons. The buttons may then be lathed into contact lenses.

Alternately, the hydrogel contact lenses may be cast directly in molds, e.g., polypropylene molds, from the mixtures, e.g., by spincasting and static casting methods. Spincasting methods are disclosed in U.S. Patent Nos. 3,408,429 and 3,660,545, and static casting methods are disclosed in U.S. Patent Nos. 4,113,224, 4,197,266, and 5,271,875. Spincasting methods involve charging the mixture mixtures to be polymerized to a mold, and spinning the mold in a controlled manner while exposing the mixture to a radiation source such as UV light. Static casting methods involve charging the monomeric mixture between two mold sections, one mold section shaped to form the anterior lens surface and the other mold section shaped to form the posterior lens surface, and curing the mixture while retained in the mold assembly to form a lens, for example, by free radical polymerization of the mixture. Examples of free radical reaction techniques to cure the lens material include thermal radiation, infrared radiation, electron beam radiation, gamma radiation, ultraviolet (UV) radiation, and the like; or combinations of such techniques may be used. U.S. Patent No. 5,271,875 describes a static cast molding method that permits molding of a finished lens in a mold cavity defined by a posterior mold and an anterior mold. As an additional method, U.S. Patent No. 4,555,732 discloses a process where an excess of a monomeric mixture is cured by spincasting in a mold to form a shaped article having an anterior lens surface and a relatively large thickness, and the posterior surface of the cured spincast article is subsequently lathed to provide a contact lens having the desired thickness and posterior lens surface.

Polymerization may be facilitated by exposing the mixture to heat and/or radiation, such as ultraviolet light, visible light, or high energy radiation. A polymerization initiator is included in the mixture to facilitate the polymerization step. Representative examples of free radical thermal polymerization initiators include organic peroxides such as acetyl peroxide, lauroyl peroxide, decanoyl peroxide, stearoyl peroxide, benzoyl peroxide, tertiarylbutyl peroxypivalate, peroxydicarbonate, and the like. Representative UV initiators are those known in the art and include benzoin methyl ether, benzoin ethyl ether, Darocure^{®} 1173, 1164, 2273, 1116, 2959, 3331 (EM Industries) and Irgacure^{®} 651 and 184 (Ciba-Geigy), and the like. Generally, the initiator will be employed in the monomeric mixture at a concentration of about 0.01 to about 5 percent by weight of the total mixture.

Polymerization is generally performed in a reaction medium, such as, for example, a solution or dispersion using a solvent, e.g., water or an alkanol containing from 1 to 4 carbon atoms such as methanol, ethanol or propan-2-ol. Alternatively, a mixture of any of the above solvents may be used.

Generally, polymerization can be carried out for about 15 minutes to about 72 hours, and under an inert atmosphere of, for example, nitrogen or argon. If desired, the resulting polymerization product can be dried under vacuum, e.g., for about 5 to about 72 hours or left in an aqueous solution prior to use.

Polymerization of the mixtures will yield a polymer, that when hydrated, forms a hydrogel. Generally, the mixture will contain the hydrophilic polymer comprising one or more hydrophilic units and one or more thio carbonyl thio fragments of a RAFT agent in an amount ranging from about 0.25 to about 15 weight percent, and preferably about 2.5 to about 7.5 weight percent, based on the total weight of the mixture. The silicone-containing comonomer may be present in the mixture in an amount ranging from about 70 to about 99 weight percent, and preferably from about 80 to about 95 weight percent, based on the total weight of the mixture.

When producing a hydrogel lens, the mixture may further include at least a diluent that is ultimately replaced with water when the polymerization product is hydrated to form a hydrogel. Generally, the water content of the hydrogel is greater than about 5 weight percent and more commonly between about 10 to about 80 weight percent. The amount of diluent used should be less than about 50 weight percent and in most cases, the diluent content will be less than about 30 weight percent. However, in a particular polymer system, the actual limit will be dictated by the solubility of the various monomers in the diluent. In order to produce an optically clear copolymer, it is important that a phase separation leading to visual opacity does not occur between the comonomers and the diluent, or the diluent and the final copolymer.

Furthermore, the maximum amount of diluent which may be used will depend on the amount of swelling the diluent causes the final polymers. Excessive swelling will or may cause the copolymer to collapse when the diluent is replaced with water upon hydration. Suitable diluents include, but are not limited to, ethylene glycol; glycerine; liquid poly(ethylene glycol); alcohols; alcohol/water mixtures; ethylene oxide/propylene oxide block copolymers; low molecular weight linear poly(2-hydroxyethyl methacrylate); glycol esters of lactic acid; formamides; ketones; dialkylsulfoxides; butyl carbitol; and the like and mixtures thereof.

If necessary, it may be desirable to remove residual diluent from the lens before edge-finishing operations which can be accomplished by evaporation at or near ambient pressure or under vacuum. An elevated temperature can be employed to shorten the time necessary to evaporate the diluent. The time, temperature and pressure conditions for the solvent removal step will vary depending on such factors as the volatility of the diluent and the specific monomeric components, as can be readily determined by one skilled in the art. If desired, the mixture used to produce the hydrogel lens may further include crosslinking and wetting agents known in the prior art for making hydrogel materials.

The hydrogel contact lenses obtained herein may be subjected to optional machining operations. For example, the optional machining steps may include buffing or polishing a lens edge and/or surface. Generally, such machining processes may be performed before or after the product is released from a mold part, e.g., the lens is dry released from the mold by employing vacuum tweezers to lift the lens from the mold, after which the lens is transferred by means of mechanical tweezers to a second set of vacuum tweezers and placed against a rotating surface to smooth the surface or edges. The lens may then be turned over in order to machine the other side of the lens.

The lens may then be transferred to individual lens packages containing a buffered saline solution. The saline solution may be added to the package either before or after transfer of the lens. Appropriate packaging designs and materials are known in the art. A plastic package is releasably sealed with a film. Suitable sealing films are known in the art and include foils, polymer films and mixtures thereof. The sealed packages containing the lenses are then sterilized to ensure a sterile product. Suitable sterilization means and conditions are known in the art and include, for example, autoclaving.

As one skilled in the art will readily appreciate other steps may be included in the molding and packaging process described above. Such other steps can include, for example, coating the formed lens, surface treating the lens during formation (e.g., via mold transfer), inspecting the lens, discarding defective lenses, cleaning the mold halves, reusing the mold halves, and the like and combinations thereof.

The following examples are provided to enable one skilled in the art to practice the invention and are merely illustrative of the invention. The examples should not be read as limiting the scope of the invention as defined in the claims.

In the examples, the following abbreviations are used.
DMA: N,N-dimethylacrylamide
HEMA: 2-hydroxyethyl methacrylate
NVP: N-vinyl-2-pyrrolidone
THF: tetrahydrofuran
ETOH: ethanol
TRIS-MA: tris(trimethylsiloxy)silylpropyl methacrylate
TRIS-VC: tris(trimethylsiloxy)silylpropyl vinyl carbamate
VazoTM 64: azo bis-isobutylnitrile (AIBN)
IMVT: 1,4-bis(4-(2-methacryloxyethyl)phenylamino)anthraquinone
Vinal Acid: Vinylcarbamate of β-Alanine having the structure: 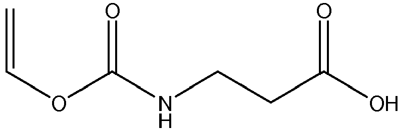
V2D25: Divinylcarbonate of PDMS diol having the structure: 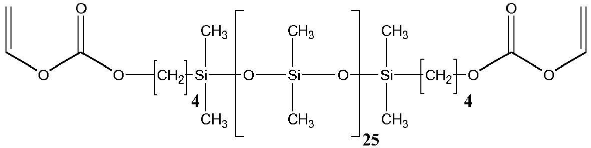
Ma2D37: Dimethacrylamide of a PDMS diamine having the structure: 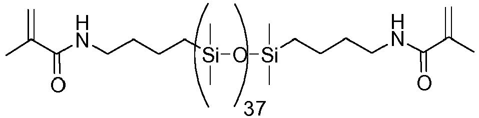
CIX-4: a compound having the structure: 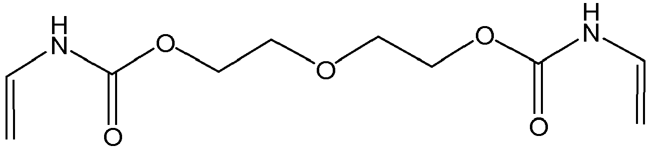
TEGDMA: a compound having the structure: 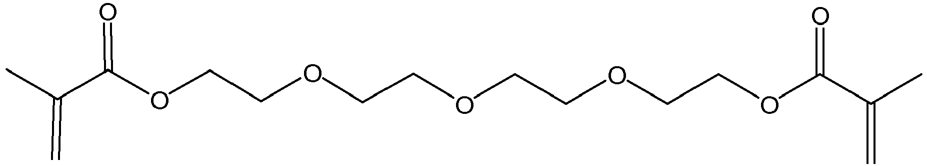
M1-MCR-C12: a compound having the structure: 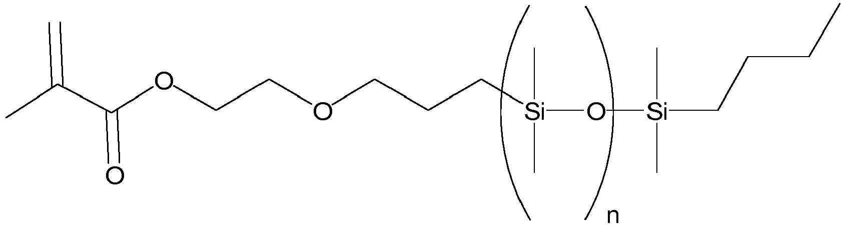
wherein n is an average of 12.

### EXAMPLE 1

### Preparation of Ethyl α-(o-ethyl xanthyl) prioprionate having the following structure:

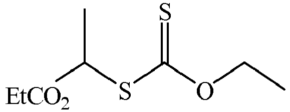

A 500 mL round bottom 3 neck flask was fitted with a magnetic stirrer, nitrogen inlet, and a temperature probe. Ethyl-2-bromo propionate (27.2 g) and 500 mL absolute ethanol were combined and stirred for 20 minutes under nitrogen. The reaction flask was placed in an ice/water bath at 0°C. Potassium O-ethyl xanthate (26.4 g) was slowly added using a powder funnel. The funnel was rinsed with an additional 50 mL of ethanol. The reaction flask was allowed to stir for an additional 24 hours at room temperature. Deionized water (250 mL) was then added to the reaction flask. The crude mixture was extracted 4 times with 200 mL of 2:1 hexane: ethyl ether retaining the organic layers. The combined organic layers were dried over sodium sulfate, filtered and solvent was removed under reduced pressure to obtain 32.22 grams of the desired product (a 97 % yield).

### EXAMPLE 2

### Preparation of α,-(Ethyl Xanthyl) Toluene having the following structure:

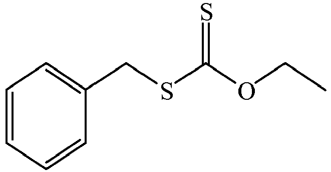

A 250 mL round bottom 3 neck flask was fitted with a magnetic stirrer, nitrogen inlet, Freidrich's condenser, and a temperature probe. After absolute ethanol (125 mL) and benzyl bromide (14.4 g) were added, the reaction flask was placed in an ice/water bath at 0°C and stirred for 1 hour. Potassium O-ethyl xanthate (17.63 g) was added slowly to the reaction flask using a powder funnel. The reaction flask was stirred for an additional 16 hours at room temperature and 200 mL of purified water was added to the flask. The crude mixture was extracted 3 times with 200 mL of 2:1 pentane:ethyl ether retaining the organic layers. The combined organic layers were dried over anhydrous sodium sulfate, filtered and solvent was removed under reduced pressure leaving 15.09 g (an 84.6% yield) of the desired product.

### EXAMPLE 3

### Preparation of (1-Phenyl Ethyl) Ethyl Xanthate having the following structure:

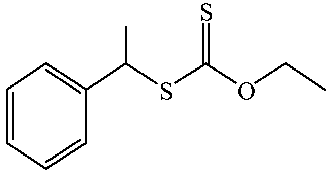

A 500 mL round bottom 3 neck flask was fitted with a magnetic stirrer, nitrogen inlet, and a temperature probe, 1-bromoethyl benzene (20.5 mL) and 200 mL absolute ethanol were added. The reaction flask was placed in an ice/water bath at 0°C. Potassium O-ethyl xanthate was added slowly using a powder funnel rinsed into the reaction flask with an additional 100 mL ethanol. The reaction flask was allowed to stir for an additional 24 hours at room temperature and then 250 mL of purified water was added. The crude mixture was extracted 4 times with 200 mL of 2:1 heptane: ethyl ether retaining the organic layers. The combined organic layers were dried over anhydrous sodium sulfate, filtered and the solvent was removed under reduced pressure to yield 31.42 grams of crude product. A portion, 15 grams, of the crude product was eluted from a silica gel column using hexane to give 12.81grams of the pure product.

### EXAMPLE 4

### Preparation of Naphthyl-O-Ethyl Xanthate having the following structure:

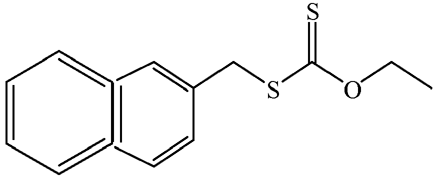

A 1000 mL round bottom 3 neck flask fitted with a mechanical stirrer, nitrogen inlet, Freidrich's condenser, and a temperature probe was charged with 500 mL of ethanol: 1,4 dioxane, and 2-(bromomethyl naphthalene) (22.1 g). The reaction flask was placed in an ice/water bath at 0°C and potassium O-ethyl xanthate (17.63 g) was added slowly using a powder funnel. The reaction stirred for an additional 16 hours at room temperature and 500 mL of puirifed water was added. The crude mixture was extracted 2 times with 500 mL of 50:50 hexane:ethyl ether, hexane, and methylene chloride retaining the organic layers. The combined organic layers were dried over anhydrous sodium sulfate, filtered and solvent was removed under reduced pressure leaving the product, a yellow oil 22.52 g (an 85.8 % yield).

### EXAMPLE 5

### Preparation of a Hydrophilic Polymer (MacroRAFT reagent).

An oven dried round bottom reaction flask fitted with a septum, magnetic stirrer and a thermo controller. The flask was charged with N-vinyl-2-pyrrolidinone (NVP) (100 grams, 0.90 mole) anhydrous 1-4 dioxane (200 ml), the RAFT reagent ethyl α-(O-ethylxanthyl)propionate of Example 1 (0.444 g, 2x10⁻³ moles) and azobisisobutrylnitrile (AIBN) (2 x 10⁻⁴ moles = 0.016g). Dry nitrogen was bubbled through the reaction mixture for 30 minutes to remove dissolved oxygen. The vessel was then heated at 60°C under nitrogen. Samples (1.5 ml) were drawn at 5, 16.5, 20, 24, 28 and 40 hours and precipitated into ethyl ether. The heat was shut off at 40 hours and the hydrophilic polymer was isolated by precipitation into a large volume (3 L) of ethyl ether. The isolated yield of the hydrophilic polymer was 71.1 grams (71 %). The results for size exclusion chromatography were Mn = 53,443 Daltons, Mw = 74,318 Daltons, Mp = 78,402 Daltons and a polydispersity of 1.39. This reaction is generally shown below in Scheme VII.

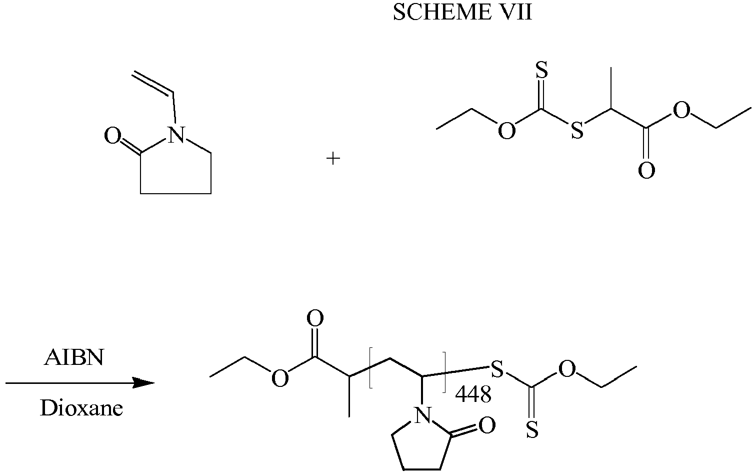

### EXAMPLES 6-27

Preparation of Hydrophilic Polymers (MacroRAFT reagents). The hydrophilic polymers of Examples 6-27 were prepared in substantially the same manner as in Example 5. The ingredients and amounts for preparing the hydrophilic polymers are set forth below in Table 1.

**TABLE 1**

| | Solvent | Monomer Information | | | RAFT Reagent | | Initiator | | Molecular Weight Data | | |
|---|---|---|---|---|---|---|---|---|---|---|---|
| Example | Volume, mL | Monomer | g | moles | Agent | g | g | Yield | Mn | Mw | PDI |
| 6 | 20 | NVP | 20.8 | 0.187 | Ex. 1 | 0.198 | 0.029 | 8.0 | 35,093 | 40,789 | 1.16 |
| 7 | 50 | NVP | 52.0 | 0.468 | Ex. 1 | 6.450 | 1.860 | 47.1 | 1,797 | 1,901 | 1.06 |
| 8 | 50 | NVP | 20.8 | 0.187 | Ex. 1 | 0.087 | 0.026 | 16.6 | 45,601 | 58,024 | 1.27 |
| 9 | 50 | NVP | 20.8 | 0.187 | Ex. 1 | 0.087 | 0.028 | 15.4 | 49,948 | 70,417 | 1.41 |
| 10 | 50 | NVP | 20.8 | 0.187 | Ex. 1 | 0.085 | 0.027 | 19.4 | 49,065 | 72,537 | 1.48 |
| 11 | 50 | NVP | 20.8 | 0.187 | Ex. 1 | 0.089 | 0.027 | 15.4 | 49,460 | 63,869 | 1.29 |
| 12 | 20 | NVP | 20.8 | 0.187 | Ex. 1 | 0.087 | 0.025 | 15.9 | 50,721 | 59,983 | 1.18 |
| 13 | 96 | NVP | 41.6 | 0.374 | Ex. 1 | 0.090 | 0.052 | 36.0 | 76,001 | 103,329 | 1.36 |
| 14 | 100 | NVP | 41.6 | 0.374 | Ex. 1 | 0.320 | 0.051 | 38.9 | 38,076 | 47,472 | 1.25 |
| 15 | 100 | NVP | 41.6 | 0.374 | Ex. 1 | 0.240 | 0.051 | 35.2 | 45,719 | 59,704 | 1.31 |
| 16 | 100 | NVP | 41.6 | 0.374 | Ex. 1 | 0.460 | 0.133 | 44.4 | 27,236 | 29,826 | 1.10 |
| 17 | 100 | NVP | 41.6 | 0.374 | Ex. 1 | 0.454 | 0.179 | 45.8 | 28,141 | 30,592 | 1.09 |
| 18 | 100 | NVP | 41.6 | 0.374 | Ex. 1 | 0.760 | 0.237 | - | 16,508 | 18,718 | 1.13 |
| 19 | 100 | NVP | 41.6 | 0.374 | Ex. 1 | 1.005 | 0.310 | - | 11,537 | 13,547 | 1.17 |
| 20 | 100 | NVP | 23.3 | 0.210 | Ex. 1 | 0.032 | 0.032 | - | 127,855 | 145,603 | 1.14 |
| 21 | 100 | NVP | 49.9 | 0.449 | Ex. 1 | 0.222 | 0.064 | 48.1 | 73,226 | 76,042 | 1.04 |
| 22 | 100 | NVP | 41.6 | 0.374 | Ex. 1 | 1.005 | 0.053 | - | 6359 | 8320 | 1.31 |
| 23 | 50 | NVP | 20.8 | 0.187 | Ex. 1 | 0.100 | 0.000 | - | 4323 | 5682 | 1.31 |
| 24 | 50 | NVP | 20.8 | 0.187 | Ex. 1 | 0.100 | 0.001 | - | 4471 | 5980 | 1.34 |
| 25 | 50 | NVP | 20.8 | 0.187 | Ex. 1 | 0.100 | 0.002 | - | 5693 | 8675 | 1.52 |
| 26 | 49 | DMA | 19.2 | 0.194 | Ex. 1 | 0.023 | 0.026 | 8.7 | 91,225 | 148,697 | 1.63 |
| 27 | 48 | DMA | 19.2 | 0.194 | Ex. 1 | 0.045 | 0.028 | 6.7 | 61,905 | 90,827 | 1.47 |

### EXAMPLE 28

### Preparation of S-sec Propionic Acid O-ethyl Xanthate.

A 1000 mL round bottom 3 neck flask was equipped with a Friedrich condenser, a magnetic stirring bar, nitrogen inlet, and a temperature probe. 2-Bromo propionic acid and 600 mL absolute ethanol were combined and stirred for 20 minutes under nitrogen. Potassium O-ethyl xanthate was added slowly using a powder funnel to the reaction flask and rinsed with an additional 50 mL of ethanol. The reaction flask was allowed to stir at a gentle reflux over night and then quenched with 250 mL of DI water. The mixture was acidified with HCl and then extracted 3 times with 250 ml portions of ether. The combined organic layers were dried over magnesium sulfate and the solvents were removed from the filtrate by flash evaporation leaving 26.3 grams of crude product a light orange liquid. This reaction is generally shown below in Scheme VIII.

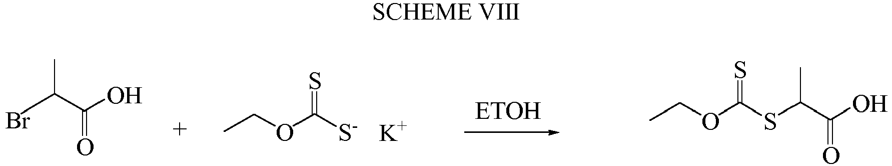

### EXAMPLE 29

### Preparation of α-Ethyl Xanthylphenylacetic Acid.

A 1000 mL round bottom 3 neck flask was fitted with a magnetic stirrer, nitrogen inlet, and a temperature probe, α-Bromophenylacetic acid (21.5g) and 300 mL ethanol were added. Potassium O-ethyl xanthate was added slowly using a powder funnel rinsed into the reaction flask with an additional 100 mL absolute ethanol. The reaction flask was allowed to stir for an additional 24 hours at 60°C and then 250 mL of purified water was added. The crude mixture was extracted 4 times with 200 mL of chloroform retaining the organic layers. The combined organic layers were dried over anhydrous sodium sulfate, filtered and the solvent was removed under reduced pressure to yield 5.18 grams the resulting product, a viscous liquid. This reaction is generally shown below in Scheme IX.

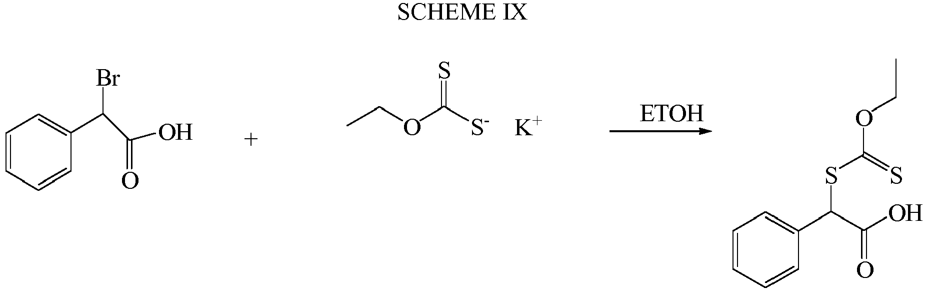

### EXAMPLE 30

### Preparation of 2(Dodecylthiocarbonylthiol)propanoic Acid.

A reaction flask was fitted a magnetic stirrer, ice bath, dropping funnel and a nitrogen inlet. The flask was charged with ethyl ether (150 ml) and 60% sodium hydride (6.3 grams). With stirring, dodecylmercaptan (30.76 grams) was slowly added to the cold slurry (temperature 5-10°C). The grayish slurry was converted to a thick white slurry (sodium thiodecylate) with vigorous evolution of H₂ gas. The mixture was cooled to 0°C and carbon disulfide (12 g) was added. Following the addition, the ice bath was removed and the reaction was allowed to reach room temperature and the addition of 2-bromopropanoic acid (23.3 grams) followed by stirring overnight. The solution was filtered to remove the salt and recrystallization from heptane gave 21 grams of pale yellow needles. This reaction is generally shown below in Scheme X.

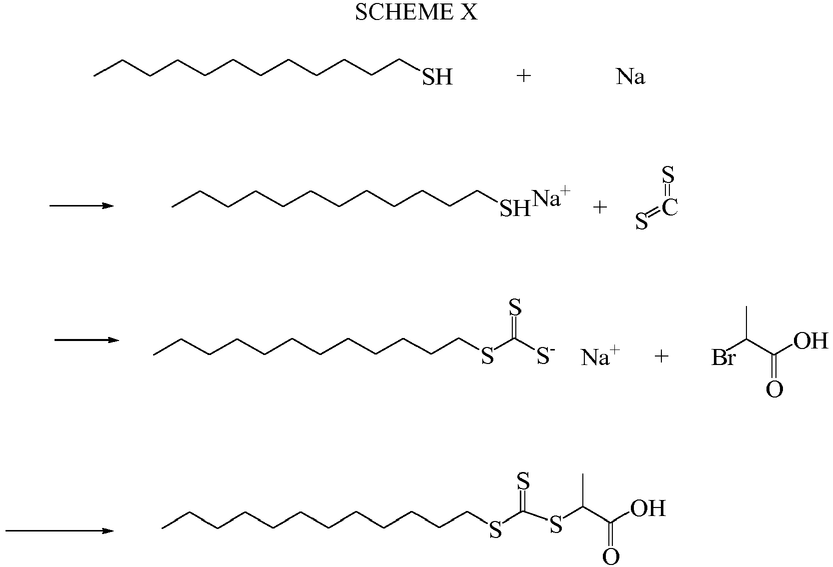

### EXAMPLE 31

### Preparation of Ethyl α-(o-ethyl xanthyl) Proprionate.

A 500 mL round bottom 3 neck flask was equipped with a Friedrich condenser, a magnetic stirring bar, nitrogen inlet, and a temperature probe. Ethyl-2-bromo propionate and 500 mL absolute ethanol were added and stirred for 20 minutes under nitrogen. The reaction flask was placed in an ice bath at 0° ± 3°C. Potassium O-ethyl xanthate was added slowly to the reaction flask using a powder funnel and rinsed with an additional 50 mL of ethanol. The reaction flask was allowed to stir and equilibrate to room temperature over a period of 24 hours. DI water (250 mL) was added to quench the reaction. The crude mixture was extracted 4 times with 200 mL of 2:1 hexane: ethyl ether retaining the organic layers. The combined organic layers were dried over sodium sulfate, filtered and solvent was removed under reduced pressure.

### EXAMPLE 32

### Preparation of Ethyl α-(ethyl xanthyl) Phenyl Acetate.

A 500 mL round bottom 3 neck flask was equipped with a magnetic stirrer, nitrogen inlet, Friedrich's condenser and a temperature probe. Ethyl (2-bromo-2-phenyl) acetate and 250 mL absolute ethanol were added and stirred for 20 minutes under nitrogen. The reaction flask was placed in an ice/water bath at 0°C. Potassium O-ethyl xanthate was added slowly using a powder funnel and rinsed into the reaction flask with an additional 50 mL of ethanol. The reaction flask was allowed to stir for an additional 24 hours at room temperature. DI water (250 mL) was then added to the reaction flask. The crude mixture was extracted 4 times with 200 mL of 2:1 hexane: ethyl ether retaining the organic layers. The combined organic layers were dried over sodium sulfate, filtered and solvent was removed under reduced pressure. Yield, 96%.

### EXAMPLE 33

### Preparation of Ethyl 2-(dodecyl trithiocarbonyl) Proprionate.

A 250 mL round bottom 3 neck flask was equipped with a mechanical stirrer, Friedrich's condenser and a temperature probe. Carbon disulfide and dodecanethiol were added to the flask with 65 mL chloroform. Triethylamine was added drop wise using an addition funnel with 10 mL chloroform. The reaction stirred for 3 hours at room temperature. Ethyl-α-bromo proprionate was added drop wise using an addition funnel with 25 mL chloroform. The reaction flask was allowed to stir for an additional 24 hrs at room temperature. The crude mixture was washed 2 times each with 250 mL of DI water, 5% HCl, and 5% Brine retaining the organic layers. The organic layers were dried over magnesium sulfate, filtered and solvent was removed under pressure. The product was further purified by column chromatography on silica gel using hexane:ethyl acetate.

### EXAMPLE 34

### Preparation of Ethyl-α-(dodecyl trithiocarbonyl) Phenyl Acetate.

A 250 mL round bottom 3 neck flask was equipped with a mechanical stirrer, Friedrich condenser and a temperature probe. Carbon disulfide and dodecanethiol were added to the flask with 65 mL chloroform. Triethylamine was added dropwise using an addition funnel with 10 mL chloroform. The reaction stirred for 3 hours at room temperature. Ethyl-α-bromophenyl acetate was added drop wise using an addition funnel with 35 mL chloroform. The reaction flask was allowed to stir for an additional 24 hours at room temperature. The crude mixture was washed 2 times with 250 mL of DI water, 5% HCl (aq), and 5% Brine retaining the organic layers. The organic layers were dried over magnesium sulfate, filtered and solvent was removed under pressure. The product was further purified by column chromatography on silica gel using hexane:ethyl acetate.

### EXAMPLE 35

### Preparation of a Hydrophilic Polymer.

An oven dried round bottom reaction flask fitted with a septum, magnetic stirrer and a thermo controller. The flask was charged with NVP, (50 grams) anhydrous 1-4 dioxane (100ml), α-ethyl xanthylphenylacetic acid of Example 29 (0.245 g, 1x10⁻³ moles) and azobisisobutrylnitrile (AIBN) (1 x 10⁻⁴ moles = 0.016 g). Dry nitrogen was bubbled through the reaction mixture for 30 minutes to remove dissolved oxygen. The vessel was then heated at 60°C under nitrogen for 14 hours. The heat was shut off at 14 hours; and allowed to cool to room temperature. The resulting hydrophilic polymer was isolated by precipitation into a large volume (3 L) of ethyl ether. The isolated yield of polymer was 21.6 grams (37 %). The results for size exclusion chromatography were Mn = 59,033 Daltons, Mw = 82,898 Daltons, Mp = 83,585 Daltons and a polydispersity of 1.40. This reaction is generally shown below in Scheme XI.

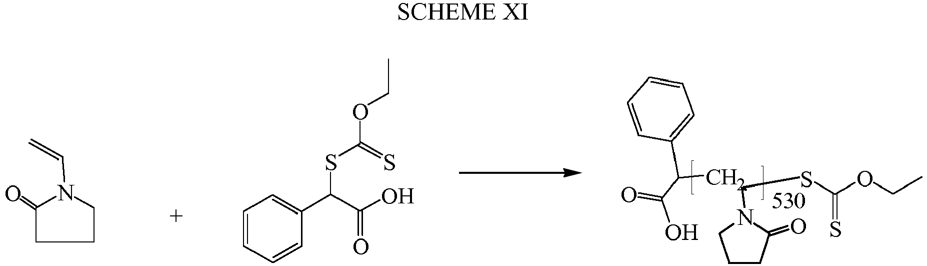

### EXAMPLE 36

### Preparation of a difunctional RAFT agent α,α'-di (ethyl xanthyl)-p-xylene having the following structure

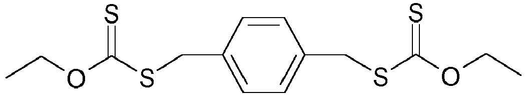

A 1000 mL round bottom 3 neck flask, fitted with a magnetic stirrer, nitrogen inlet, and a temperature probe, was charged with α,α'-diBr p-xylene (0.150 moles, 39.6 grams), absolute ethanol (ETOH,125 ml) and anhydrous tetrahydrofuran (THF,125 ml). The reaction flask was cooled in an ice bath and half of potassium O-ethyl xanthate (KEX, 0.165 moles, 26.4g) was slowly added through a powder funnel followed by an additional 250 ml of ETOH/THF [1:1]. This was followed by a second addition of the remaining KEX (0.165 moles, 26.4g) and an additional 300 ml of solvent mixture. Once the additions were complete, the reaction mixture was stirred for 24 hours at room temperature. Purified water (250 ml was then added to the reaction. The mixture was extracted four times with hexane (250 ml). The organic layers were combined, dried over anhydrous sodium sulfate and filtered. The product was concentrated by flash evaporation at reduced pressure.

### EXAMPLE 37

### Preparation of a Hydrophilic Polymer (MacroRAFT reagent).

An oven dried round bottom reaction flask, fitted with a septum, magnetic stirrer and a thermo controller, was charged with NVP, (20.8 grams) anhydrous 1-4 dioxane (50 ml), α,α'-di (ethyl xanthyl)-p- xylene of Example 36 (0.134 g, 4x10⁻⁴ moles) and azobisisobutrylnitrile (AIBN) (3.1 x 10⁻⁴ moles = 0.051 g). Dry nitrogen was bubbled through the reaction mixture for 30 minutes to remove dissolved oxygen. The vessel was then heated at 60°C under nitrogen for 14 hours. The heat was shut off at 14 hours; and allowed to cool to room temperature. The resulting hydrophilic polymer was isolated by precipitation into a large volume of ethyl ether. The isolated yield of the hydrophilic polymer was 6.05 grams (29 %). The results for size exclusion chromatography were Mn = 21,723 Daltons, Mw = 24,771 Daltons, and a polydispersity of 1.14.

### EXAMPLE 38

### Preparation of a Hydrophilic Polymer (MacroRAFT reagent).

An oven dried round bottom reaction flask fitted with a septum, magnetic stirrer and a thermo controller. The flask was charged with NVP, (41.6 grams) anhydrous 1-4 dioxane (100 ml), α,α'-di (ethyl xanthyl)-p-xylene of Example 36 (0.267 g, 8x10⁻⁴ moles) and azobisisobutrylnitrile (AIBN) (3.36 x 10⁻⁴ moles = 0.0552 g). Dry nitrogen was bubbled through the reaction mixture for 30 minutes to remove dissolved oxygen. The vessel was then heated at 60°C under nitrogen for 14 hours. The heat was shut off at 14 hours; and allowed to cool to room temperature. The resulting hydrophilic polymer was isolated by precipitation into a large volume of ethyl ether. The isolated yield of the hydrophilic polymer was 45.34 grams. The results for size exclusion chromatography were Mn = 47,333 Daltons, Mw = 65,372 Daltons, and a polydispersity of 1.38

### EXAMPLES 39-49 AND COMPARATIVE EXAMPLES A AND B

Preparation of Contact Lenses, Films, and Flats. The amounts and ingredients for each of the formulations of Comparative Example A and Examples 8-10 are set forth below in Table 2.

**TABLE 2**

| Ex./ Comp. Ex. | RAFT Agent | RAFT (parts) | V2D25 (parts) | NVP (parts) | TRIS-VC (parts) | Vinal Acid (parts) | D1173 (parts) | Diluent (parts) |
|---|---|---|---|---|---|---|---|---|
| 39 | Ex. 21 | 6.9 | 13.8 | 27.5 | 50.5 | 0.92 | 0.46 | 13.8 (H) |
| 40 | Ex. 20 | 6.9 | 13.8 | 27.5 | 50.5 | 0.92 | 0.46 | 13.8 (H) |
| 41 | Ex. 17 | 9.0 | 13.5 | 26.9 | 49.3 | 0.90 | 0.45 | 13.5 (H) |
| 42 | Ex. 18 | 9.0 | 13.5 | 26.9 | 49.3 | 0.90 | 0.45 | 13.5 (H) |
| 43 | Ex. 19 | 9.0 | 13.5 | 26.9 | 49.3 | 0.90 | 0.45 | 13.5 (H) |
| 44 | Ex. 11 | 9.0 | 13.5 | 26.9 | 49.3 | 0.90 | 0.45 | 13.5 (H) |
| 45 | Ex. 19 | 9.0 | 13.5 | 26.9 | 49.3 | 0.90 | 0.45 | 13.5 (H) |
| 46 | Ex. 21 | 6.9 | 13.8 | 27.5 | 50.5 | 0.92 | 0.46 | 13.8 (H) |
| 47 | Ex. 21 | 6.9 | 13.8 | 27.5 | 50.5 | 0.92 | 0.46 | 13.8 (N) |
| 48 | Ex. 21 | 6.9 | 13.8 | 27.5 | 50.5 | 0.92 | 0.46 | 13.8 (N) |
| 49 | Ex. 18 | 9.0 | 13.5 | 26.9 | 49.3 | 0.90 | 0.45 | 13.5 (N) |
| A | - | - | 14.8 | 29.6 | 54.2 | 0.99 | 0.49 | 14.8 (H) |
| B | - | - | 14.8 | 29.6 | 54.2 | 0.99 | 0.49 | 14.8 (H) |

Contact lenses, films and lens flats were prepared in polypropylene molds. Flat thicknesses of 300, 450, 550 and 650 µm were cast in order to obtain the oxygen permeability of the materials. Lens samples were cast to determine water content, modulus, tensile strength, percent elongation and tear strength.

In the casting procedure, all mold parts were placed in a nitrogen chamber at least 18 hours prior to casting. The anterior mold was filled with the specified volume of the mixture and then capped with a posterior mold half. The filling and capping procedure was carried out under nitrogen. The capped molds were placed in a holding plate and transferred to a nitrogen purged oven where they were cured by exposure to UV light under a continuous nitrogen purge for 1-2 hours at ambient temperature or 55°C. Molds were separated manually and the lenses were released in a 30% solution of isopropyl alcohol/water overnight. The lenses were extracted by swelling in 100% isopropyl alcohol for four hours. The isopropyl alcohol concentration was reduced to 50% with water and then the lenses were stepped into 100% water.

Films were cast between 3.5 x 4 inch silane treated glass plates separated by Teflon gaskets ranging from 0.2 to 1 mm in thickness. Films were cured under UV light at 55°C or ambient temperature for 1-2 hours. Films were extracted by swelling in 100 % isopropyl alcohol for four hours. The isopropyl alcohol concentration was reduced to 50% with water and then samples were stepped into 100% water. Film samples were used for mechanical testing and water content measurements.

### Physical Properties

The techniques used for determining the physical properties for the lenses, films and flats of Examples 39-40 and Comparative Examples A and B are described below.

Water %: Two sets of six hydrated lenses or films are blotted dry on a piece of filter paper to remove excess water, and samples are weighed (wet weight). Samples are then placed in a microwave oven for 10 minutes inside ajar containing dessicant. The samples are then allowed to sit for 30 minutes to equilibrate to room temperature and reweighed (dry weight). The percent water is calculated from the wet and dry weights.

Mechanical properties: Modulus and elongation tests were conducted according to ASTM D-1708a, employing an Instron (Model 4502) instrument where the hydrogel film sample is immersed in borate buffered saline; an appropriate size of the film sample is gauge length 22 mm and width 4.75 mm, where the sample further has ends forming a dogbone shape to accommodate gripping of the sample with clamps of the Instron instrument. All results are based on the average thickness at the center. The standard deviation is given in parenthesis.

Oxygen permeability: Dk was determined by the following procedure. Other methods and/or instruments may be used as long as the oxygen permeability values obtained therefrom are equivalent to the described method. The oxygen permeability of silicone hydrogels is measured by the polarographic method (ANSI Z80.20-1998) using an 02 Permeometer Model 201T instrument (Createch, Albany, California USA) having a probe containing a central, circular gold cathode at its end and a silver anode insulated from the cathode. Measurements are taken only on pre-inspected pinhole-free, flat silicone hydrogel film samples of four different center thicknesses ranging from 150 to 600 microns. Center thickness measurements of the film samples may be measured using a Rehder ET-1 electronic thickness gauge.

Generally, the film samples have the shape of a circular disk. Measurements are taken with the film sample and probe immersed in a bath containing circulating phosphate buffered saline (PBS) equilibrated at 35°C+/- 0.2°. Prior to immersing the probe and film sample in the PBS bath, the film sample is placed and centered on the cathode premoistened with the equilibrated PBS, ensuring no air bubbles or excess PBS exists between the cathode and the film sample, and the film sample is then secured to the probe with a mounting cap, with the cathode portion of the probe contacting only the film sample. For silicone hydrogel films, it is frequently useful to employ a Teflon polymer membrane, e.g., having a circular disk shape, between the probe cathode and the film sample. In such cases, the Teflon membrane is first placed on the pre-moistened cathode, and then the film sample is placed on the Teflon membrane, ensuring no air bubbles or excess PBS exists beneath the Teflon membrane or film sample.

Once measurements are collected, only data with correlation coefficient value (R2) of 0.97 or higher should be entered into the calculation of Dk value. At least two Dk measurements per thickness, and meeting R2 value, are obtained. Using known regression analyses, oxygen permeability (Dk) is calculated from the film samples having at least three different thicknesses. Any film samples hydrated with solutions other than PBS are first soaked in purified water and allowed to equilibrate for at least 24 hours, and then soaked in PHB and allowed to equilibrate for at least 12 hours. The instruments are regularly cleaned and regularly calibrated using RGP standards. Upper and lower limits are established by calculating a +/- 8.8% of the Repository values established by William J. Benjamin, et al., The Oxygen Permeability of Reference Materials, Optom Vis Sci 7 (12s): 95 (1997), the disclosure of which is incorporated herein in its entirety:

| Material Name | Repository Values | Lower Limit | Upper Limit |
|---|---|---|---|
| Fluoroperm 30 | 26.2 | 24 | 29 |
| Menicon EX | 62.4 | 56 | 66 |
| Quantum II | 92.9 | 85 | 101 |

The corresponding physical properties are set forth below in Table 3.

**TABLE 3**

| Ex./ Comp. Ex. | Water (%) | Dk | Modulus | Tear | Tensile | Elong., | Clarity | F=film, Fl=flat L=lens |
|---|---|---|---|---|---|---|---|---|
| 39 | 43.6 | ND | 69 (5) | 4 (0.4) | 41 (10) | 120 (24) | - | F, Fl |
| 41 | 46.2 | 69 | 62 (10) | 4 (1) | 31 (15) | 90 (42) | - | F |
| 42 | 46.4 | 63 | 58 (9) | 4 (0.2) | 38 (18) | 119 (54) | - | F |
| 43 | 47.9 | 67 | 55 (7) | 4 (1) | 27 (13) | 101 (48) | - | F |
| 45 | 47.0 | 93 | 71 (14) | 4 (1) | 53 (18) | 119 (44) | Clear | F, Fl, L |
| 46 | 35.2 | 103 | 223 (11) | 4 (0.5) | 110 (29) | 82 (15) | Clear | F, Fl, L |
| 47 | 44.2 | 82 | 109 (10) | 4 (2) | 100 (34) | 140 (22) | Clear | F, Fl |
| 48 | 44.2 | 89 | 122 (10) | 3 (1) | 76 (37) | 114 (46) | Cloudy | F, Fl, L |
| 49 | 44.6 | 101 | 112 (14) | 3 (0.2) | 63 (15) | 98 (25) | Cloudy | F, Fl, L |
| A | 36.0 | 84 | 147 (16) | 6 (0.4) | 74 (27) | 122 (35) | - | F, Fl |
| B | 48.0 | 70 | 70 (4) | 3 (1) | 40 (18) | 103 (49) | Clear | F, Fl, L |

### EXAMPLES 50-55

### Preparation of Hydrophilic Polymer (Poly DMA-co-mPEG 1000 MacroRAFT reagent).

For Example 53, into a one neck, 250mL round bottom flask equipped with a magnetic stirring bar was added 392 mg (0.891 mmol) of ethyl α-dodecyltrithiocarbonyl phenyl acetate (EDTCPA), 43 mL of DMA (41.4 g, 0.4173 moles), 4.629 g of monomethoxy polyethylene glycol 1000 methacrylate (mPEG) (4.21 mmol) and 100mL of anhydrous 1,4-dioxane. After these components were thoroughly mixed, one mL of an 8.59 mM AIBN solution in 1,4-dioxane (1,41mg/mL) was then carefully pipetted into the flask. The round bottom flask was closed with an appropriately sized rubber septum and the contents of the flask were then purged by bubbling dry nitrogen gas for 1 hour. The contents of the reaction flask were heated to 60°C with an oil bath for 18 hours, cooled to room temperature, and precipitated dropwise into 2500 mL of ether while stirring vigorously. The polymer product was then filtered and dried under vacuum to remove residual ether.

Examples 54 through 58 were carried out in the same manner as Example 53 except using varying amounts of mPEG and DMA and the type of mPEG. The RAFT agent utilized in each of the examples was EDTCPA in approximately 0.390 g for each example. The amounts of the ingredients are set forth below in Table 4.

**TABLE 4**

| Ex. | mPEG (g) | DMA (g) | mPEG | % Yield | Yield (g) | GPC Data | | |
|---|---|---|---|---|---|---|---|---|
| | | | | | | Mw | Mn | PDI |
| 50 | 4.63 | 41.4 | 1000 | 81.0 | 37.3 | 55400 | 42400 | 1.31 |
| 51 | 2.3 | 20.0 | 1000 | 97.5 | 21.7 | 32000 | 26000 | 1.23 |
| 52 | 0.2 | 20.7 | 400 | 74.3 | 15.6 | 29400 | 24400 | 1.20 |
| 53 | 2.0 | 43 | 400 | 79.5 | 34.5 | 52200 | 37900 | 1.38 |
| 54 | 2.0 | 41.4 | 400 | 83.6 | 36.3 | 60800 | 51900 | 1.17 |
| 55 | 2.0 | 41.4 | 400 | 80.3 | 34.9 | 61600 | 45800 | 1.35 |

### EXAMPLES 56-58 AND COMPARATIVE EXAMPLES C-E

Preparation of biomedical devices using the poly(DMA-co-mPEG) hydrophilic polymer of Example 53. The amounts and ingredients for each of the formulations of Examples 56-58 and Comparative Examples C-E are set forth below in Table 5.

**TABLE 5**

| | Comp. Ex. C | Ex. 56 | Comp. Ex. D | Ex. 57 | Comp. Ex. E | Ex. 58 |
|---|---|---|---|---|---|---|
| TRIS-MA | 31.7 | 24.2 | 31.7 | 36.1 | 31.6 | 36.0 |
| NVP | 32.1 | 36.1 | 0.0 | 0.0 | 0.0 | 0.0 |
| DMA | 0.0 | 0.0 | 32.3 | 24.2 | 32.2 | 24.3 |
| CIX-4 | 0.3 | 0.3 | 0.0 | 0.0 | 0.3 | 0.3 |
| M1-MCR-Cl2 | 23.2 | 22.2 | 23.3 | 22.3 | 23.2 | 22.2 |
| TEGDMA | 3.2 | 3.2 | 3.2 | 3.2 | 3.2 | 3.2 |
| Ex. 53 | 0.0 | 5.0 | 0.0 | 5.2 | 0.0 | 5.0 |
| HEMA | 9.2 | 8.7 | 9.2 | 8.7 | 9.2 | 8.7 |
| Darocure 1173 | 0.3 | 0.3 | 0.3 | 0.3 | 0.3 | 0.3 |
| Hexanol | 41.0 | 42.5 | 41.1 | 40.8 | 40.9 | 40.8 |
| Total | 141.0 | 142.5 | 141.1 | 140.8 | 140.9 | 140.8 |

Contact lenses and lens flats were prepared in substantially the same manner as in Examples 39-49. Lens flats were cast in order to obtain the oxygen permeability of the materials. Contact lenses were cast to determine water content, modulus, tensile strength, percent elongation and tear strength.

### Physical Properties

The physical properties of Examples 56-58 and Comparative Examples C-E are set forth below in Table 6.

**TABLE 6**

| | Comp. Ex. C | Ex. 56 | Comp. Ex. D | Ex. 57 | Comp. Ex. E | Ex. 58 |
|---|---|---|---|---|---|---|
| % Water | 39.2 | 55.3 | 32.6 | 34.5 | 31.0 | 36.3 |
| Dk | 94.0 | 90.0 | 100.0 | 64.0 | 105.0 | 92.0 |
| Modulus | 93 (9) | 47 (8) | 53 (4) | 34 (4) | 72 (5) | 47 (3) |
| Tensile | 45 (21) | 29 (15) | 30 (20) | 27 (11) | 44 (15) | 34 (10) |
| % Elongation | 77 (42) | 75 (34) | 98 (65) | 123 (45) | 115 (40) | 111 (30) |
| Tear | 5 (0.2) | 3 (1) | 4 (1) | 8 (0.2) | 3 (0.5) | 4 (1) |
| COF Static | 10.25 | 1.61 | ND | ND | ND | ND |
| COF Kinetic | 1.91 | 1.08 | ND | ND | ND | ND |
| Advancing Contact Angle | 90 | 47 | 90 | 47 | 90 | 48 |
| Receding Contact Angle | 29 | 27 | 31 | 30 | 32 | 31 |
| Hysteresis | 60 | 20 | 59 | 17 | 58 | 17 |

As can be seen from the data, the biomedical devices of Examples 56-58 containing a poly(DMA-co-mPEG) polymer possessed a higher water content, surface wettability (advancing contact angle and hysteresis), and lubricity (as measured using Coefficient of Friction) and a lower modulus as compared to the biomedical devices of Comparative Examples C-E.

The methods used for coefficient of friction and contact angle are briefly described below:
Coefficient of Friction: Tribological testing was performed on a CETR Model UMT-2 micro-tribometer. Each lens was clamped on an HDPE holder that initially mates with the posterior side of the lens. A poly(propylene) clamping ring was then used to hold the edge region of the lens. Once the lens was mounted in the holder the assembly was placed in a stationary clamping device within the micro-tribometer. A polished stainless steel disc containing 1mL of phosphate buffered saline (PBS) was then brought into contact with the lens and F_{N} was adjusted to 2 grams over the course of the run for the frictional measurements. After the load equilibrated for 5 seconds the stainless steel disc was rotated at a velocity of 12cm/sec for a duration of 20 seconds in both the forward and reverse directions and the peak (static) and average (kinetic) COF values were recorded. Each value represents the average of 6 lenses. All data was normalized to the average values obtained at 2g force from the lens holder in the absence of a lens tested in PBS. PBS was used as the test-in solution for every lens.

Captive Bubble Contact Angle: Captive bubble contact angle data was collected on a First Ten Angstroms FTA-1000 Drop Shape Instrument. All samples were rinsed in HPLC grade water prior to analysis in order to remove components of the packaging solution from the sample surface. Prior to data collection the surface tension of the water used for all experiments was measured using the pendant drop method. In order for the water to qualify as appropriate for use, a surface tension value of 70 - 72 dynes/cm was expected. All lens samples were placed onto a curved sample holder and submerged into a quartz cell filled with HPLC grade water. Advancing and receding captive bubble contact angles were collected for each sample. The advancing contact angle is defined as the angle measured in water as the air bubble is retracting from the lens surface (water is advancing across the surface). All captive bubble data was collected using a high speed digital camera focused onto the sample/air bubble interface. The contact angle was calculated at the digital frame just prior to contact line movement across the sample/air bubble interface. The receding contact angle is defined as the angle measured in water as the air bubble is expanding across the sample surface (water is receding from the surface).

### EXAMPLES 59-61

### Preparation of Hydrophilic Polymer (PDMA-MacroRAFT reagent).

S-1-Dodecyl-S-(α,□α'-dimethyl-α"-acetic acid) trithiocarbonate (DDAATC) and AIBN were added to a 250 ml round bottom flask. Next, DMA and 1,4-dioxane were added to the flask. The flask was sealed with a septum and then purged with argon to deoxygenate for 30 minutes. The flask was placed in a 50°C oil bath for 2 hours. After 2 hours, the reaction cooled to room temperature and precipitated into 2.5 L of diethyl ether. The polymer was isolated by filtration and dried in vacuum oven to constant weight. The amount of the ingredients for each of Examples 59-61 are set forth below in Table 7.

**TABLE 7**

| Ex. | DMA (mL) | DDAATC (mg) | Dioxane (mL) | AIBN (mg) | Theoretical Mol. Wt. |
|---|---|---|---|---|---|
| 59 | 20 | 350 | 60 | 6.8 | 19800 |
| 60 | 20 | 175 | 60 | 6.8 | 39600 |
| 61 | 20 | 700 | 60 | 13.1 | 9900 |

### EXAMPLES 62 and 63

### Preparation of Hydrophilic Polymer (PVP-MacroRAFT reagent).

AIBN was added to a 500 mL round bottom flask equipped with a magnetic stirring bar. Next, ethyl-α-(O-ethylxanthyl) propionate (EEXP), 1,4-dioxane, and NVP were added to the flask. The flask was then sealed with a rubber septum and purged with N₂ for 30 minutes. The flask was placed in an oil bath (60°C) for 16 hours. After cooling to room temperature, the contents of the flask were precipitated into 4L of diethyl ether. The precipitate was isolated by filtration and dried in vacuo to provide the PVP MacroRAFT agent. The amount of the ingredients for each of Examples 62 and 63 are set forth below in Table 8.

**TABLE 8**

| Ex. | NVP (g) | EEXP (mg) | Dioxane (mL) | AIBN (mg) | Theoretical Mol. Wt. |
|---|---|---|---|---|---|
| 62 | 80 | 2 | 220 | 445 | 8880 |
| 63 | 120 | 1.12 | 320 | 164 | 23900 |

### COMPARATIVE EXAMPLES F-H

### Removal of Trithiocarbonate End group

To remove the RAFT end group, 4.0 grams of the hydrophilic polymer from Example 59 (PDMA MacroRAFT agent) was dissolved in 15 mL of dioxane in a round bottom flask. To the flask was added 250 microliters of tris(trimethylsilyl) silane and 65.8 mgs of AIBN. The solution was sparged with nitrogen for 30 minutes and then heated at 80 °C for 12 h under a nitrogen blanket. The cooled solution was precipitated by dropwise addition into diethyl ether. The white solid was collected by vacuum filtration and vacuum dried at RT. Cleavage of the trithiocarbonate end group was evidenced by the loss of yellow color of the product and the disappearance of the dodecyl resonances in the proton NMR. Examples 65 and 66 were carried out in substantially the same manner using the hydrophilic polymer from Examples 60 and 61, respectively.

### COMPARATIVE EXAMPLES I-J

### Removal of Xanthate End group

To remove the RAFT end group, 10.0 grams of the hydrophilic polymer from Example 62 (PVP MacroRAFT agent) was dissolved in 40 mL of 1,4-dioxane in a round bottom flask. To the flask was added 690 microliters of tris(trimethylsilyl) silane and 183 mgs of AIBN. The solution was sparged with nitrogen for 30 minutes and then heated at 80 °C for 12 h under a nitrogen blanket. The cooled solution was precipitated by dropwise addition into diethyl ether. The white solid was collected by vacuum filtration and vacuum dried at room temperature. Example 68 was carried out in substantially the same manner using the hydrophilic polymer from Example 63.

### EXAMPLES 64-68 AND COMPARATIVE EXAMPLES K-O

### Preparation of biomedical devices.

Formulations were prepared in which 0.3 g of the uncleaved hydrophilic polymers from Examples 59-63 and the cleaved hydrophilic polymers of Comparative Examples F-J were dissolved in 1.7 g of the mixture obtained in Comparative Example A (see Table 2). Films of all of these formulations were cast between glass plates and cured in the UV oven for 2 hours. Films were removed from glass slides and placed in the vacuum oven at 80°C for 2 hours and at room temperature. Films were cut into pieces for percent extractables and % water determination. For percent extractables, films were weighed, extracted in 20 mL of isopropyl alcohol overnight, decanted off the isopropyl alcohol and dried sample in a vacuum oven before reweighing. For % water, additional samples were extracted in isopropyl alcohol overnight and then exchanged with deinonized water several times before being weighed in its hydrated state, dried in the vacuum oven and reweighed. Results for this study are set forth below in Table 9.

**TABLE 9**

| Ex./ Comp. Ex. | Test | Uncleaved | Cleaved | Polymer used (Examples) | Polymer |
|---|---|---|---|---|---|
| Ex. 64, Comp. Ex. K | % Water | 40.5 | 36.3 | Ex. 59, Comp. Ex. F | PDMA |
| | % Extractables | 25.7 | 22.6 | | MW=19,800 |
| Ex. 65, Comp. Ex. L | % Water | 41.9 | 38.3 | Ex. 60, Comp. Ex. G | PDMA |
| | % Extractables | 21.4 | 21.5 | | MW=39,600 |
| Ex. 66, Comp. Ex. M | % Water | 40.6 | 36.7 | Ex. 61, Comp. Ex. H | PDMA |
| | % Extractables | 26.0 | 23.9 | | MW=9,900 |
| Ex. 67, Comp. Ex. N | % Water | 43.3 | 38.9 | Ex. 62, Comp. Ex. I | PVP |
| | % Extractables | 24.7 | 20.8 | | MW=8880 |
| Ex. 68, Comp. Ex. O | % Water | 44.0 | 42.4 | Ex. 63, Comp. Ex. J | PVP |
| | % Extractables | 24.6 | 23.3 | | MW=23,900 |

As can be seen in Table 9, for lenses prepared using the uncleaved PDMA MacroRAFT reagent and the uncleaved PVP MacroRAFT reagent, the water contents and the percent extractables were higher as compared to the lenses prepared with the cleaved RAFT reagents. The higher water contents can be attributed to the higher incorporation rate of the hydrophilic MacroRAFT reagents due to their ability to covalently couple to the network by actively participating in the free radical reaction. The higher percent extractables can also be explained by the end group (trithiocarbonate or xanthate) slowing the polymerization of the network down (confirmed by DSC measurements) and leading to higher residual monomer left at the end of the polymerization. This example shows the advantageous effect of having the thio carbonyl thio group on the polymer during the polymerization.

### EXAMPLES 69-75

### Preparation of Random Copolymers.

An oven dried round bottom reaction flask fitted with a septum, magnetic stirrer and a thermo controller. The flask was charged with monomer 1, monomer 2, anhydrous 1-4 dioxane, RAFT reagent ethyl α-(O-ethylxanthyl)propionate (EEXP), and AIBN (1.52 x 10⁻⁴ moles = 0.025g). Dry nitrogen was bubbled through the reaction mixture for 30 minutes to remove dissolved oxygen. The vessel was then heated at 60°C under a passive blanket of nitrogen overnight. The copolymer with a RAFT end group was isolated by precipitation into a large volume (3 L) of ethyl ether.. The reagents and amounts for each example are set forth below in Table 10.

**TABLE 10**

| Ex. | Solvent | | Monomer | | Co-monomer | | RAFT Reagent | | Yield |
|---|---|---|---|---|---|---|---|---|---|
| | Solvent | Volume (mL) | Monomer 1 | (g) | Monomer 2 | (g) | Agent | (g) | |
| 69 | 1,4 Dioxane | 100 | NVP | 42.7 | Allyl alcohol | 1.23 | EEXP | 0.179 | 39.29 |
| 70 | 1,4 Dioxane | 100 | NVP | 42.5 | Allyl alcohol | 2.47 | EEXP | 0.172 | 40.46 |
| 71 | 1,4 Dioxane | 100 | NVP | 42.6 | Allyl alcohol | 3.92 | EEXP | 0.171 | 41.35 |
| 72 | 1,4 Dioxane | 100 | DMA | 38.5 | HEMA | 2.6 | EEXP | 0.182 | 23.87 |
| 73 | 1,4 Dioxane | 100 | DMA | 38.5 | HEMA | 5.481 | EEXP | 0.174 | 32.36 |
| 74 | 1,4 Dioxane | 100 | DMA | 38.5 | HEMA | 8.74 | EEXP | 0.170 | 35.29 |
| 75 | 1,4 Dioxane | 100 | NVP | 41.6 | Allyl alcohol | 12.9 | EEXP | 0.176 | |

The random copolymers of Examples 69-75 had the following characteristics as set forth below in Table 11.

**TABLE 11**

| Ex. | Mn, (calcd) | Mol. Weight Data | | | |
|---|---|---|---|---|---|
| | | Method | Mn | Mw | Polydisp. |
| 69 | 54,942 | SEC | 25,388 | 46,847 | 1.845 |
| 70 | 58,231 | SEC | 20,221 | 40,538 | 2.005 |
| 71 | 60,720 | SEC | 15,780 | 29,578 | 1.874 |
| 72 | 50,352 | SEC | 58,994 | 88,356 | 1.498 |
| 73 | 56,373 | SEC | 71,275 | 117,864 | 1.654 |
| 74 | 61,859 | SEC | 104,779 | 188,244 | 1.797 |
| 75 | 69,240 | SEC | 4,200 | 8,915 | 2.123 |

It will be understood that various modifications may be made to the embodiments disclosed herein. Therefore the above description should not be construed as limiting, but merely as exemplifications of preferred embodiments. For example, the functions described above and implemented as the best mode for operating the present invention are for illustration purposes only. Other arrangements and methods may be implemented by those skilled in the art without departing from the scope of this invention.

## Claims

1. A hydrogel contact lens formed from a free radical polymerization product of a mixture comprising (a) one or more hydrophilic polymers comprising one or more hydrophilic units and one or more thio carbonyl thio fragments of a reversible addition fragmentation chain transfer ("RAFT") agent; and (b) one or more silicone-containing monomers, and (c) a polymerisation initiator, wherein the hydrophilic units are derived from a hydrophilic monomer selected from the group consisting of an unsaturated carboxylic acid, acrylamide, vinyl lactam, poly(alkyleneoxy)(meth)acrylate, (meth)acrylic acid, hydroxyl-containing-(meth)acrylate, hydrophilic vinyl carbonate, hydrophilic vinyl carbamate monomer, hydrophilic oxazolone monomer, and mixtures thereof and
wherein the one or more thio carbonyl thio fragments is of a RAFT agent having the general formula 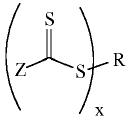 wherein x is 1 or 2, Z is a substituted oxygen, a substituted nitrogen, a substituted sulfur, a substituted or unsubstituted C₁-C₂₀ alkyl or C₃-C₂₅ unsaturated, or partially or fully saturated ring or a carboxylic acid-containing group; and R is independently a straight or branched, substituted or unsubstituted C₁-C₃₀ alkyl group, a substituted or unsubstituted C₃-C₃₀ cycloalkyl group, a substituted or unsubstituted C₃-C₃₀ cycloalkylalkyl group, a substituted or unsubstituted C₃-C₃₀ cycloalkenyl group, a substituted or unsubstituted C₅-C₃₀ aryl group, a substituted or unsubstituted C₅-C₃₀ arylalkyl group, a C₁-C₂₀ ester group; an ether or polyether-containing group; an alkyl- or arylamide group; an alkyl- or arylamine group; a substituted or unsubstituted C₅-C₃₀ heteroaryl group; a substituted or unsubstituted C₃-C₃₀ heterocyclic ring; a substituted or unsubstituted C₄-C₃₀ heterocycloalkyl group; a substituted or unsubstituted C₆-C₃₀ heteroarylalkyl group; and combinations thereof.

2. The hydrogel contact lens according to Claim 1, wherein the one or more thio carbonyl thio fragments is of a RAFT agent comprising a dithioester group, xanthate group, dithiocarbamate group or trithiocarbonate group.

3. The hydrogel contact lens according to Claims 1 and 2, wherein the RAFT agent comprises a carboxylic acid-containing group.

4. The hydrogel contact lens according to Claims 1-3, wherein the RAFT agent comprises a carboxylic acid-containing group.

5. The hydrogel contact lens according to Claims 1-4, wherein the one or more thio carbonyl thio fragments is of a RAFT agent selected from the group consisting of benzyl dodecyl trithiocarbonate, ethyl-2-(dodecyl trithiocarbonyl) proprionate, S-sec propionic acid O-ethyl xanthate, α-ethyl xanthylphenylacetic acid, ethyl α-(o-ethyl xanthyl) proprionate, ethyl α-(ethyl xanthyl) phenyl acetate, ethyl 2-(dodecyl trithiocarbonyl) phenyl acetate, ethyl 2-(dodecyl trithiocarbonyl) propionate, 2-(dodecylthiocarbonylthiol)propanoic acid and mixtures thereof.

6. The hydrogel contact lens according to Claims 1-5, wherein the hydrophilic units comprise between 10 and about 3000 units.

7. The hydrogel contact lens according to Claims 1-6, wherein the one or more hydrophilic polymers further comprise units derived from an ethylenically unsaturated polymerizable alkoxylated polymer is selected from the group consisting of polyethylene glycol (PEG)-200 methacrylate, PEG-400 methacrylate, PEG-600 methacrylate, PEG-1000 methacrylate and mixtures thereof.

8. The hydrogel contact lens according to Claims 1-7, wherein the one or more hydrophilic polymers is a random or block copolymer.

9. The hydrogel contact lens according to Claims 1-8, wherein the mixture further comprises a hydrophilic monomer, hydrophobic monomer or both.

10. The hydrogel contact lens according to Claims 1-9, wherein the one or more silicone-containing monomers is a hydrophilic monomer or hydrophobic monomer.

## Patentansprüche

1. Eine Hydrogelkontaktlinse hergestellt aus einem Produkt einer freien radikalischen Polymerisation eines Gemisches umfassend (a) ein oder mehrere hydrophile(s) Polymer(e) umfassend eine oder mehrere hydrophile Einheit(en) und ein oder mehrere Thiocarbonylthio-Fragment(e) eines reversiblen Addition-Fragmentierung-Kettenübertragungs-Mittels (RAFT) und (b) ein oder mehrere silikonhaltige(s) Monomer(e) und (c) einen Polymerisationsinitiator, wobei die hydrophilen Einheiten von einem hydrophilen Monomer ausgewählt aus der Gruppe bestehend aus ungesättigter Carboxylsäure, Acrylamid, Vinyllactam, Poly(alkylenoxy)(meth)acrylat, (Meth)acrylsäure, hydroxylhaltigem (Meth)acrylat, hydrophilem Vinylcarbonat, hydrophilem Vinylcarbamatmonomer, hydrophilem Oxazolonmonomer und Gemischen davon erhalten werden und
wobei das eine oder die mehreren Thiocarbonylthio-Fragment(e) eines RAFT-Mittels die folgende allgemeine Formel aufweisen 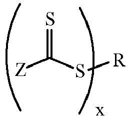 wobei x 1 oder 2 ist, Z ein substituierter Sauerstoff, ein substituierter Stickstoff, ein substituierter Schwefel, eine substituiertes oder unsubstituiertes C₁-C₂₀ Alkyl oder ein C₃-C₂₅ ungesättigter oder teilweise oder komplett gesättigter Ring oder eine carboxylsäurehaltige Gruppe ist und R unabhängig voneinander eine geradkettige oder verzweigte, substituierte oder unsubstituierte C₁-C₃₀ Alkylgruppe, eine substituierte oder unsubstituierte C₃-C₃₀ Cycloalkylgruppe, eine substituierte oder unsubstituierte C₃-C₃₀ Cycloalkylalkylgruppe, eine substituierte oder unsubstituierte C₃-C₃₀ Cycloalkenylgruppe, eine substituierte oder unsubstituierte C₅-C₃₀ Arylgruppe, eine substituierte oder unsubstituierte C₅-C₃₀ Arylalkylgruppe, eine C₁-C₂₀ Estergruppe, eine ether- oder polyetherhaltige Gruppe, eine Alkyl- oder Arylamidgruppe, eine Alkyl- oder Arylamingruppe, eine substituierte oder unsubstituierte C₅-C₃₀ Heteroarylgruppe, ein substituierter oder unsubstituierter C₃-C₃₀ heterocyclischer Ring, eine substituierte oder unsubstituierte C₄-C₃₀ Heterocyclolalkylgruppe, eine substituierte oder unsubstituierte C₆-C₃₀ Heteroarylalkylgruppe oder Kombinationen davon ist.

2. Die Hydrogelkontaktlinse gemäß Anspruch 1, wobei das eine oder die mehreren Thiocarbonylthio-Fragment(e) des RAFT-Mittels eine Dithioestergruppe, Xanthatgruppe, Dithiocarbamatgruppe oder Trithiocarbonatgruppe umfasst/umfassen.

3. Die Hydrogelkontaktlinse gemäß Ansprüchen 1 oder 2, wobei das RAFT-Mittel eine carboxylsäurehaltige Gruppe umfasst.

4. Die Hydrogelkontaktlinse gemäß Ansprüchen 1-3, wobei das RAFT-Mittel eine carboxylsäurehaltige Gruppe umfasst.

5. Die Hydrogelkontaktlinse gemäß Ansprüchen 1-4, wobei das eine oder die mehreren Thiocarbonylthio-Fragment(e) eines RAFT-Mittels ausgewählt ist/sind aus der Gruppe bestehend aus Benzyldodecyltrithiocarbonat, Ethyl-2-(dodecyltrithiocarbonyl)proprionat, S-sec-Propionsäure-O-ethylxanthat, α-Ethylxanthylphenylessigsäure, Ethyl-α-(o-Ethylxanthyl)proprionat, Ethyl-α-(Ethylxanthyl)phenylacetat, Ethyl-2-(Dodecyltrithiocarbonyl)phenylacetat, Ethyl-2-(Dodecyltrithiocarbonyl)propionat, 2-(Dodecylthiocarbonylthiol)propanonsäure und Gemischen davon.

6. Die Hydrogelkontaktlinse gemäß Ansprüchen 1-5, wobei die hydrophilen Einheiten zwischen 10 und ungefähr 3000 Einheiten umfassen.

7. Die Hydrogelkontaktlinse gemäß Ansprüchen 1-6, wobei das eine oder die mehreren hydrophilen Polymer(e) weiterhin Einheiten erhalten von einem ethylenisch ungesättigten polymerisierbaren alkoxylierten Polymer ausgewählt aus der Gruppe bestehend aus Polyethylenglycol (PEG)-200 Methacrylat, PEG-400 Methacrylat, PEG-600 Methacrylat, PEG-1000 Methacrylat und Gemischen davon umfasst/umfassen.

8. Die Hydrogelkontaktlinse gemäß Ansprüchen 1-7, wobei das eine oder die mehreren hydrophile(n) Polymer(e) ein Random- oder Blockcopolymer ist/sind.

9. Die Hydrogelkontaktlinse gemäß Ansprüchen 1-8, wobei das Gemisch weiterhin ein hydrophiles Monomer, hydrophobes Monomer oder beide umfasst.

10. Die Hydrogelkontaktlinse gemäß Ansprüchen 1-9, wobei das eine oder die mehreren silikonhaltige(n) Monomer(e) ein hydrophiles Monomer oder hydrophobes Monomer ist / sind.

## Revendications

1. Lentille de contact hydrogel formée à partir d'un produit de polymérisation de radicaux libres provenant d'un mélange comprenant (a) un ou plusieurs polymères hydrophiles comprenant une ou plusieurs unités hydrophiles et un ou plusieurs fragments thiocarbonylthio d'un agent de transfert de chaîne à fragmentation-addition réversible (« RAFT ») ; et (b) un ou plusieurs monomères contenant du silicium, et (c) un initiateur de polymérisation, dans lequel les unités hydrophiles sont dérivées à partir d'un monomère hydrophile choisi parmi le groupe constitué d'un acide carboxylique insaturé, d'un acrylamide, d'une lactame de vinyle, d'un poly(méth)acrylate d'alkylèneoxy, d'un acide (méth)acrylique, d'un (méth)acrylate contenant de l'hydroxyle, d'un carbonate de vinyle hydrophile, d'un monomère de carbamate de vinyle hydrophile, d'un monomère d'oxazolone hydrophile, et des mélanges de ceux-ci et
dans laquelle l'un ou plusieurs des fragments thiocarbonylthio provient d'un agent RAFT possédant la formule générale 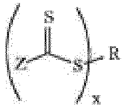 dans laquelle X vaut 1 ou 2, Z représente un oxygène substitué, un azote substitué, un sulfure substitué, un alkyle en C₁-C₂₀ substitué ou non substitué ou un noyau en C₃-C₂₅ non saturé, ou partiellement ou entièrement saturé substitué ou non substitué, ou un groupe contenant un acide carboxylique ; et R représente indépendamment un groupe alkyle en C₁-C₃₀ substitué ou non substitué, linéaire ou ramifié, un groupe cycloalkyle en C₃-C₃₀ substitué ou non substitué, un groupe groupe cycloalkylalkyle en C₃-C₃₀ substitué ou non substitué, un groupe cycloalcényle en C₃-C₃₀ substitué ou non substitué, un groupe aryle en C₅-C₃₀ substitué ou non substitué, un groupe arylalkyle en C₅-C₃₀ substitué ou non substitué, un groupe ester en C₄-C₂₀ ; un éther ou un groupe contenant un polyester ; un groupe alkylamide ou arylamide ; un groupe alkylamine ou arylamine ; un groupe hétéroaryle en C₅-C₃₀ substitué ou non substitué ; un noyau hétérocyclique en C₃-C₃₀ substitué ou non substitué ; un groupe hétérocycloalkyle en C₄-C₃₀ substitué ou non substitué ; un groupe hétéroalkylalkyle en C₆-C₃₀ substitué ou non substitué ; et des combinaisons de ceux-ci.

2. Lentille de contact hydrogel selon la revendication 1, dans laquelle l'un ou plusieurs des fragments thiocarbonylthio provient d'un agent RAFT comprenant un groupe dithioester, un groupe xanthate, un groupe dithiocarbamate ou un groupe trithiocarbonate.

3. Lentille de contact hydrogel selon la revendication 1 et 2, dans laquelle l'agent RAFT comprend un groupe contenant un acide carboxylique.

4. Lentille de contact hydrogel selon l'une quelconque des revendications 1 - 3, dans laquelle l'agent RAFT comprend un groupe contenant un acide carboxylique.

5. Lentille de contact hydrogel selon l'une quelconque des revendications 1 - 4, dans laquelle l'un ou plusieurs des fragments thiocarbonylthio provient d'un agent RAFT choisi parmi le groupe constitué du dodécyltrithiocarbonate de benzyle, de l'éthyl-2-(dodécyltrithiocarbonyl)propionate, de l'O-éthylxanthate de l'acide S-sec propionique, de l'acide xanthylphénylacétique α-éthylique, de l'α-(o-éthylxanthyl)propionate d'éthyle, de l'α-(éthylxanthyl)phénylacétate d'éthyle, du 2-(dodécyltrithiocarbonyl)phénylacétate d'éthyle, du 2-(dodécyltrithiocarbonyl)propionate d'éthyle, de l'acide 2-(dodécylthiocarbonylthiol)propanoïque et des mélanges de ceux-ci.

6. Lentille de contact hydrogel selon l'une quelconque des revendications 1 - 5, dans laquelle les unités hydrophiles comprennent entre 10 et environ 3000 unités.

7. Lentille de contact hydrogel selon l'une quelconque des revendications 1 - 6, dans laquelle l'un ou plusieurs des polymères hydrophiles comprend en outre des unités dérivées d'un polymère alcoxylé polymérisable et insaturé de manière éthylènique choisi depuis le groupe constitué du méthacrylate de polyéthylène glycol (PEG)-200, du méthacrylate de PEG-400, du méthacrylate de PEG-600, du méthacrylate de PEG-1000 et des mélanges de ceux-ci.

8. Lentille de contact hydrogel selon l'une quelconque des revendications 1 - 7, dans laquelle l'un ou plusieurs des polymères hydrophiles est un copolymère statistique ou séquencé.

9. Lentille de contact hydrogel selon l'une quelconque des revendications 1 - 8, dans laquelle le mélange comprend en outre un monomère hydrophile, un monomère hydrophobe ou les deux.

10. Lentille de contact hydrogel selon l'une quelconque des revendications 1 - 9, dans laquelle l'un ou plusieurs des monomères contenant du silicium est un monomère hydrophile ou un monomère hydrophobe.
